# EUROPEAN PATENT APPLICATION

(11) **EP 4 660 146 A1**
(43) Date of publication of application: **10.12.2025**
(21) Application number: 24750420.2
(22) Date of filing: 02.02.2024
(51) Int. Cl.: C01G 27/02

(54) **HAFNIUM-ACID-COMPOUND-CONTAINING MATERIAL AND METHOD FOR PRODUCING SAME**

(30) Priority: 03.02.2023 JP 2023015419
(71) Applicant: Mitsui Mining & Smelting Co., Ltd, Shinagawa-ku Tokyo 141-8584 (JP)
(72) Inventor: ARAKAWA, Daiki, Omuta-shi, Fukuoka 836-0003 (JP); SEKI SATO, Riko, Omuta-shi, Fukuoka 836-0003 (JP); HARA, Shuhei, Omuta-shi, Fukuoka 836-0003 (JP)
(74) Representative: Novagraaf Technologies
(86) International application number: PCT/JP2024/003544
(87) International publication number: WO 2024/162471

(57) **Abstract**

A hafnium polyoxometalate compound-containing material of the present invention contains hafnium, an organic nitrogen compound, and a solvent, in which the ratio of an XRD maximum intensity (Ib) at a diffraction angle 2θ of 5° or more and 6° or less to an XRD maximum intensity (Ia) at a diffraction angle 2θ of 31° or more and 33° or less, Ib/Ia, in dried powder obtained by drying the hafnium polyoxometalate compound-containing material at 150°C for 15 hours in a vacuum atmosphere, is 1.2 or more. The hafnium polyoxometalate compound-containing material of the present invention contains hafnium, an organic nitrogen compound, and a solvent, in which a maximum value of transmittance in a wavelength region of 550 nm to 700 nm is 70%T or more.

## Description

### Technical Field

The present invention relates to a hafnium polyoxometalate compound-containing material and a method for producing the same.

### Background Art

Hafnium oxide, for example, hafnium oxide (IV) and compounds thereof have a high relative permittivity characteristic, and thus have been used as a gate insulator for transistors and the like. As a ferroelectric material, hafnium oxide (IV) is expected to allow ferroelectric memories to have an extremely small thickness and a low voltage. Further, hafnium oxide (IV), which has a very high melting point, thus has been used for insulating heat-resistance materials such as thermocouples. For example, Patent Literature 1 discloses a crystalline hafnium oxide sol containing powdered hafnium oxide (IV), that is, hafnium powder. The crystalline hafnia sol is particulate and monodispersed, and thus can be used as a raw material for refractories, insulators, derivatives and the like.

### Citation List

### Patent Literature

Patent Literature 1: JP 2007-223881 A

### Summary of Invention

### Technical Problem

However, the crystalline hafnia sol disclosed in Patent Literature 1 is an acidic sol containing nitric acid, and therefore is difficult to combine with a liquid containing another basic metal element or the like, and unsuitable for use with an oxoacid or halogen ions, or the like in combination and film formation.

In view of the problems described above, the present invention provides a basic and highly dispersive hafnium polyoxometalate compound-containing material, and a method for producing the same.

### Solution to Problem

A hafnium polyoxometalate compound-containing material of the present invention which has been made to solve the above-described problems is a hafnium polyoxometalate compound-containing material containing hafnium, an organic nitrogen compound and a solvent, in which a ratio of an XRD maximum intensity (Ib) at a diffraction angle 2θ of 5° or more and 6° or less to an XRD maximum intensity (Ia) at a diffraction angle 2θ of 31° or more and 33° or less, Ib/Ia, in dried powder obtained by drying the hafnium polyoxometalate compound-containing material at 150°C for 15 hours in a vacuum atmosphere is 1.1 or more.

From the viewpoint of excellent dispersibility, the hafnium polyoxometalate compound-containing material of the present invention is preferably a hafnium polyoxometalate compound-containing material containing hafnium, an organic nitrogen compound and a solvent, in which the ratio of an XRD maximum intensity (Ib) at a diffraction angle 2θ of 5° or more and 6° or less to an XRD maximum intensity (Ia) at a diffraction angle 2θ of 31° or more and 33° or less, Ib/Ia, in dried powder obtained by drying the hafnium polyoxometalate compound-containing material at 150°C for 15 hours in a vacuum atmosphere is 1.1 or more.

It is presumed that hafnium in the hafnium polyoxometalate compound-containing material of the present invention is present in the containing material as ions obtained by multistage condensation of hafnium atoms and oxygen atoms in the containing material.

The hafnium polyoxometalate compound-containing material of the present invention contains an organic nitrogen compound in addition to hafnium. The "organic nitrogen compound" according to the present invention includes those ionized in the hafnium polyoxometalate compound-containing material of the present invention. As will be described later in detail for a method for producing a hafnium polyoxometalate compound-containing material according to the present invention, in a relevant production process, a hydrous hafnium acid ammonium cake, which is a hafnium-containing precipitation slurry, is formed by a reverse neutralization method in which an acidic hafnium solution is added to aqueous ammonia, an organic nitrogen compound is then added, and mixing is performed to form the hafnium polyoxometalate compound-containing material of the present invention. Therefore, a substituted organic nitrogen compound may be present as a cation in the containing material.

Examples of the solvent for the hafnium polyoxometalate compound-containing material of the present invention include water and organic solvents. Examples of the organic solvent include alcohol solvents, ketone solvents, ether solvents, ester solvents, aromatic hydrocarbon solvents, and aliphatic hydrocarbon solvents. The organic solvent may be a solvent obtained by mixing any these organic solvents with water. Examples of the alcohol solvent include alcohols having 5 or less carbon atoms (methanol, ethanol, n-propanol, isopropyl alcohol, butanol, ethylene glycol, propylene glycol and glycerin), and acetone.

In the hafnium polyoxometalate compound-containing material of the present invention, the ratio of an XRD maximum intensity (Ib) at a diffraction angle 2θ of 5° or more and 6° or less to an XRD maximum intensity (Ia) at a diffraction angle 2θ of 31° or more and 33° or less, Ib/Ia, in dried powder obtained by drying the hafnium polyoxometalate compound-containing material at 150°C for 15 hours in a vacuum atmosphere, is preferably 1.1 or more, and more preferably 1.2 or more, from the viewpoint of excellent solubility of the hafnium polyoxometalate compound in a solvent.

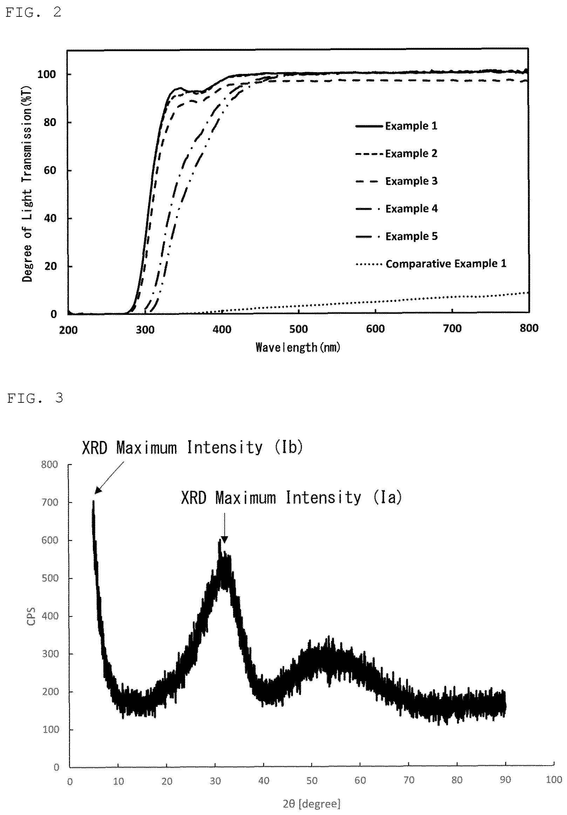

Here, commonly distributed hafnium oxide has a monoclinic crystal structure at ordinary temperature and ordinary pressure, whose XRD spectrum has two peaks with a maximum intensity in the vicinity of a diffraction angle 2θ of 28° to 32° according to the card number: 00-034-0104 Quality: S. On the other hand, as shown in Fig. 3, the XRD spectrum of dried powder obtained by drying the hafnium polyoxometalate compound-containing material of the present invention as described above has a unique pattern with a maximum intensity in the vicinity of a diffraction angle 2θ of 5° to 6°, which is shaper than the pattern with a maximum intensity in the vicinity of a diffraction angle 2θ of 31° to 33°. Therefore, the hafnium polyoxometalate compound-containing material is thought to have a crystal structure different from the commonly distributed hafnium oxide, for example, a plurality of crystal structures including an amorphous structure. The hafnium polyoxometalate compound-containing material of the present invention is presumed to have better solubility in a solvent over commonly distributed hafnium oxide when the ratio of the XRD maximum intensity (Ib) to the XRD maximum intensity (Ia), Ib/Ia, in the dried powder is 1.1 or more, and more preferably 1.2 or more.

The XRD maximum intensity (Ia) and the XRD maximum intensity (Ib) in the hafnium polyoxometalate compound-containing material of the present invention can be measured by taking 100 g of the hafnium polyoxometalate compound-containing material of the present invention in a 100 mL Teflon (registered trademark) beaker, drying the hafnium polyoxometalate compound-containing material at 150°C for 15 hours in a vacuum atmosphere, and subjecting the thus-obtained dried powder to the following X-ray powder diffraction measurement conditions. Subsequently, a ratio of the XRD maximum intensity (Ib) to the XRD maximum intensity (Ia), Ib/Ia, in the dried powder is calculated.

### = X-ray powder diffraction measurement conditions =

- Diffraction apparatus: MiniFlex2 (manufactured by Rigaku Corporation)
- X-ray tube: Cu
- Tube voltage/tube current: 30 kV, 15 mA
- Slit: DS-SS; 1.25 degrees, RS; 0.3 mm
- Monochromator graphite
- Measurement interval: 0.01 degrees
- Counting method: Fixed time mode
- X-ray analysis software: PDXL2 Version2.9.1.0

When the hafnium polyoxometalate compound-containing material of the present invention is dried in a vacuum atmosphere to obtain dried powder, the crystal phase of the obtained dried powder changes depending on the heating temperature. For example, when the heating temperature is 150°C, the crystal structure of the dried powder includes a plurality of crystal structures including an amorphous structure, whereas when the hafnium polyoxometalate compound-containing material is fired at a heating temperature of, for example, 800°C in an air atmosphere and then allowed to cool to room temperature (25°C), the crystal structures of the dried powder are integrated into a monoclinic crystal.

A change in crystal phase in the hafnium polyoxometalate compound-containing material of the present invention can also be observed by the full width at half maximum of the XRD maximum intensity (Ia). In the hafnium oxide having a monoclinic crystal structure, the full width at half maximum of the XRD maximum intensity (Ia) is likely to be small because of the single crystal structure. On the other hand, in the dried powder, the crystal structure changes, and the full width at half maximum tends to be large.

The term "containing material" in the present invention refers to a liquid form described below or a liquid form containing a gel, and the liquid form is not limited to solutes dispersed or mixed in a single molecule state in a solvent, and also includes aggregates in which a plurality of molecules attract one another through intermolecular interaction, for example, (1) multimer molecules, (2) solvate molecules, (3) molecular clusters and (4) colloidal particles dispersed in a solvent.

The hafnium polyoxometalate compound-containing material of the present invention is a hafnium polyoxometalate compound-containing material containing hafnium, an organic nitrogen compound, and a solvent, in which a maximum value of transmittance in a wavelength region of 550 nm to 700 nm is 70%T or more.

From the viewpoint of excellent dispersibility, the hafnium polyoxometalate compound-containing material of the present invention is preferably a hafnium polyoxometalate compound-containing material containing hafnium, an organic nitrogen compound, and a solvent, in which the maximum value of transmittance in a wavelength region of 550 nm to 700 nm is 70%T or more.

The hafnium polyoxometalate compound-containing material of the present invention contains hafnium, an organic nitrogen compound, and a solvent as described above.

Further, in the hafnium polyoxometalate compound-containing material of the present invention, the maximum value of transmittance in a wavelength region of 550 nm to 700 nm is preferably 70%T or more from the viewpoint of excellent solubility of the hafnium polyoxometalate compound in a solvent. The maximum value of transmittance in a wavelength region of 550 nm to 700 nm is more preferably 75%T or more, still more preferably 80%T or more, and particularly preferably 85%T or more, more preferably 90%T or more, still more preferably 95%T or more, or particularly preferably 98%T or more, more preferably 99%T or more, and most preferably 100%T or more. If the measured value exceeds 100%T due to a measurement error of a spectrophotometer used for measuring the transmittance, the transmittance is regarded as 100%T.

In the hafnium polyoxometalate compound-containing material of the present invention, at least one of the transmittances at wavelengths of 550 nm, 600 nm, 650 nm and 700 nm is preferably 70%T or more, more preferably 75%T or more, still more preferably 80%T or more, and particularly preferably 85%T or more, more preferably 90%T or more, still more preferably 95%T or more, or particularly preferably 98%T or more, more preferably 99%T or more, and most preferably 100%T or more. If the measured value exceeds 100%T due to a measurement error of a spectrophotometer used for measuring the transmittance, the transmittance is regarded as 100%T.

Further, in the hafnium polyoxometalate compound-containing material of the present invention, the minimum value of transmittances in a wavelength range of 550 nm to 700 nm is preferably 70%T or more, more preferably 75%T or more, still more preferably 80%T or more, and particularly preferably 85%T or more, more preferably 90%T or more, still more preferably 95%T or more, or particularly preferably 98%T or more, more preferably 99%T or more, and most preferably 100%T or more. If the measured value exceeds 100%T due to a measurement error of a spectrophotometer used for measuring the transmittance, the transmittance is regarded as 100%T.

A liquid in which the maximum value of transmittance of the hafnium polyoxometalate compound-containing material of the present invention in a wavelength region of 400 nm to 700 nm is 70%T or more is defined as a "hafnium polyoxometalate compound-containing material" of the present invention. In the present specification, unless otherwise specified, the term "transmittance" includes both of "initial transmittance" indicating the transmittance of the hafnium polyoxometalate compound-containing material of the present invention adjusted to a liquid temperature of 25°C immediately after it is formed, and "transmittance after lapse of time" indicating the transmittance of the hafnium polyoxometalate compound-containing material after it is left standing for 1 month in a thermostat set at a room temperature of 25°C, from the day on which the hafnium polyoxometalate compound-containing material of the present invention is formed.

The hafnium polyoxometalate compound-containing material of the present invention adjusted to room temperature (25°C) is placed in a quartz cell having an optical path length of 5.0 mm, and the transmittance of the containing material in a wavelength range of 550 nm to 700 nm is measured according to JIS K0115, 2004 "General rules for molecular absorptiometric analysis" using a spectrophotometer under the following transmittance measurement conditions.

### = transmittance measurement conditions =

- Measuring apparatus: Ultraviolet-visible near-infrared spectrophotometer model UH4150 (manufactured by Hitachi High-Tech Science Corporation)
- Measurement mode: Wavelength scan
- Data mode: %T (transmission)
- Measurement wavelength range: 200 to 2,600 nm
- Scan speed: 600 nm/min
- Sampling interval: 2 nm

In the hafnium polyoxometalate compound-containing material of the present invention, the hafnium content in the hafnium polyoxometalate compound-containing material is more than 0 to 10 mass% in terms of HfO₂.

In the hafnium polyoxometalate compound-containing material of the present invention, the hafnium content in the hafnium polyoxometalate compound-containing material is preferably more than 0 to 10 mass% in terms of HfO₂ from the viewpoint of improving the stability of the containing material. The hafnium content in the hafnium polyoxometalate compound-containing material is more preferably 0.1 to 10 mass% in terms of HfO₂.

Here, the hafnium content in the hafnium polyoxometalate compound-containing material of the present invention is calculated by appropriately diluting the containing material with dilute hydrochloric acid as necessary, and measuring the Hf weight fraction in terms of hafnium oxide (HfO₂) according to JIS K0116:2014 by highfrequency inductively coupled plasma emission spectrometry (ICP emission spectrometry (AG-5110 manufactured by Agilent Technologies)). Hafnium in the hafnium polyoxometalate compound-containing material of the present invention is not necessarily present in the form of HfO₂. The indication of the hafnium content in terms of HfO₂ is based on the conventional way of indicating a hafnium polyoxometalate content.

The hafnium polyoxometalate compound-containing material of the present invention may contain hydrogen peroxide in addition to the above-described hafnium, organic nitrogen compound and solvent.

The "hydrogen peroxide" according to the present invention includes those ionized in the hafnium polyoxometalate compound-containing material of the present invention. As will be described later in detail for a method for producing a hafnium polyoxometalate compound-containing material according to the present invention, in a relevant production process, addition of hydrogen peroxide to an acidic hafnium solution may complex anionic species including hafnium is complexed, resulting in formation of a peroxo complex having excellent dissolution stability.

In the quantitative analysis of hydrogen peroxide present in the containing material, for example, hydrogen peroxide present in the containing material is quantitated by measuring the absorbance of the containing material at a wavelength of 410 nm using a spectrophotometer (U-2900 manufactured by Hitachi, Ltd.).

The hafnium polyoxometalate compound-containing material of the present invention may contain water in addition to the above-described hafnium and organic nitrogen compound.

Since the hafnium polyoxometalate compound in the hafnium polyoxometalate compound-containing material of the present invention has high dispersibility in water and good solubility in water, pure water can be used as the solvent.

In the hafnium polyoxometalate compound-containing material of the present invention, the organic nitrogen compound is quaternary ammonium.

The organic nitrogen compound is preferably quaternary ammonium from the viewpoint of exhibiting not only high solubility but also suppressing crystallization to a high degree and suppressing solation to a high degree.

Examples of the quaternary ammonium include alkylimidazolium, pyridinium, pyrrolidium, and tetraalkylammonium. Here, specific examples of the alkylimidazolium include 1-methyl-3-methylimidazolium, 1-ethyl-3-methylimidazolium, 1-propyl-3-methylimidazolium, 1-butyl-3-methylimidazolium, 1-hexyl-3-methylimidazolium, 1-methyl-2,3-dimethylimidazolium, 1-ethyl-2,3-dimethylimidazolium, 1-propyl-2,3-dimethylimidazolium, and 1-butyl-2,3-dimethylimidazolium. Specific examples of pyridinium and pyrrolidinium include N-butyl-pyridinium, N-ethyl-3-methyl-pyridinium, N-butyl-3-methyl-pyridinium, N-hexyl-4-(dimethylamino)-pyridinium, N-methyl-1-methylpyrrolidinium, and N-butyl-1-methylpyrrolidinium. Further, specific examples of tetraalkylammonium include tetramethylammonium, tetraethylammonium, tetrabutylammonium, ethyl-dimethyl-propylammonium, and choline. Examples of the anion that forms a salt with the cation include OH⁻, Cl⁻, Br⁻, I⁻, BF₄⁻, and HSO₄⁻.

Examples of the method for measuring the content of the organic nitrogen compound present in the containing material include gas chromatography (GC), liquid chromatography (LC), mass spectrometry (MS), gas chromatography/mass spectrometry (GC-MS), and liquid chromatography/mass spectrometry (LC-MS), and a method of quantitating N₂ in the gasified sample with a thermal conductivity meter may be used in combination.

In the hafnium polyoxometalate compound-containing material of the present invention, the quaternary ammonium is one or more selected from tetramethylammonium hydroxide (TMAH), tetraethylammonium hydroxide (TEAH), choline, and benzyltrimethylammonium hydroxy.

The quaternary ammonium is preferably one or more selected from TMAH, TEAH, choline and benzyltrimethylammonium hydroxy.

The quaternary ammonium may be a mixture of two or more, instead of one, selected from TMAH, TEAH, choline and benzyltrimethylammonium hydroxy. Specific examples thereof include a mixture of two kinds of quaternary ammonium such as TMAH and TEAH, TMAH and choline, TMAH and benzyltrimethylammonium hydroxy, a mixture of three kinds of quaternary ammonium such as TMAH, TEAH and choline, and a mixture of four kinds of quaternary ammonium such as TMAH, TEAH, choline and benzyltrimethylammonium hydroxy.

In the hafnium polyoxometalate compound-containing material of the present invention, the pH of the hafnium polyoxometalate compound-containing material is preferably more than 7.

This is because when the pH of the hafnium polyoxometalate compound-containing material of the present invention is more than 7, the containing material is further excellent in stability. The pH is more preferably 10 or more, the pH is still more preferably 11 or more, the pH is particularly preferably 12 or more, the pH is more particularly preferably 13 or more, and the pH may be 14 or more. In the present specification, unless otherwise specified, the term "pH" refers to "initial pH" which is the pH of the hafnium polyoxometalate compound-containing material of the present invention adjusted to a liquid temperature of 25°C immediately after it is formed, as well as "pH after lapse of time" which is the pH of the hafnium polyoxometalate compound-containing material after it is left standing for 1 month in a thermostat set at a room temperature of 25°C, from the day on which the hafnium polyoxometalate compound-containing material of the present invention is formed.

Here, the pH of the hafnium polyoxometalate compound-containing material of the present invention is measured after an electrode (standard ToupH electrode 9615S-10D manufactured by HORIBA, Ltd.) of a pH meter (glass electrode type hydrogen ion concentration indicator D-51 manufactured by HORIBA, Ltd.) is immersed in the hafnium polyoxometalate compound-containing material of the present invention and it is confirmed that the liquid temperature has stabilized at 25°C.

The hafnium polyoxometalate compound-containing material of the present invention may further contain a resin.

It is preferable that the hafnium polyoxometalate compound-containing material of the present invention contains a resin because the resin uniformly blends with the hafnium polyoxometalate compound, and functions to adhere to a substrate, resulting in improvement of film formability and adhesion with respect to the substrate.

Examples of the resin contained in the hafnium polyoxometalate compound-containing material include polyolefin-based compounds and polyvinyl-based compounds.

Further, the resin contained in the hafnium polyoxometalate compound-containing material of the present invention may be an anionic water-soluble resin or a nonionic water-soluble resin.

When the resin contained in the hafnium polyoxometalate compound-containing material of the present invention is an anionic water-soluble resin and/or a nonionic water-soluble resin, the anionic water-soluble resin and/or the nonionic water-soluble resin uniformly blend with the hafnium polyoxometalate compound described above and function to adhere to the substrate, resulting in improvement of film formability and adhesion to the plastic film substrate.

Here, the cationic water-soluble resin is a resin having, in a polymer, a functional group which has a positive electric charge in water with a pH of 7 and which is, for example, any of an amino group, a tertiary amine group, a quaternary ammonium group and a hydrazino group. The anionic water-soluble resin is a resin having, in a polymer, a functional group which has a negative electric charge in water with a pH of 7 and which is, for example, any of a carboxyl group, a sulfone group, a sulfate group and a phosphate group. Further, the nonionic water-soluble resin is a resin which does not correspond to the above-described cationic water-soluble resin or anionic water-soluble resin and which has, for example, any functional group selected from a hydroxy group, an ether group and an amide group, in a polymer.

Further, the resins preferably contain one or more water-soluble homopolymers selected from the group consisting of an acrylic polymer, a urethane polymer, a styrene polymer, an olefin polymer, an amide polymer, a siloxane polymer, an epoxy polymer, a vinyl chloride polymer, and a vinyl acetate polymer, and/or water-soluble copolymers of two or more of these polymers. In particular, the resins preferably contain one or more water-soluble homopolymers selected from an acrylic polymer, a styrene polymer and an olefin polymer, and/or water-soluble copolymers of two or more of these polymers.

The hafnium polyoxometalate compound-containing material of the present invention may further contain a high-boiling-point solvent. Here, the high-boiling-point solvent is preferably a solvent having a boiling point of 180°C or higher at 1 atm, and examples thereof include polyhydric alcohol-based solvents and glycol-based solvents.

Here, examples of the polyhydric alcohol-based solvent include glycerin (boiling point: 290°C), 1,6 hexanediol (boiling point: 250°C), and 1,7 heptanediol (boiling point: 259°C). The glycol-based solvent may be ethylene glycol (boiling point: 197.3°C), propylene glycol (boiling point: 188.2°C), diethylene glycol (boiling point: 244.3°C), triethylene glycol (boiling point: 287.4°C), oligoethylene glycol (boiling point: 287°C to 460°C), polyethylene glycol (PEG) (boiling point: 460°C or higher), polyethylene glycol (PEG)-polypropylene glycol (PPG) copolymer (boiling point: 460°C or higher), diethylene glycol monohexyl ether (boiling point: 260°C), polyoxyalkylene monoalkyl ether (boiling point: 260°C or higher), and polyoxyethylene sorbitan monolaurate (boiling point: 321°C or higher), as well as anionic fluorine-based surfactants (boiling point: 180°C or higher), amphoteric fluorine-based surfactants (boiling point: 180°C or higher), nonionic fluorine-based surfactants (boiling point: 180°C or higher), and amine oxide (boiling point: 180°C or higher). Glycerin is particularly preferable. The above-described boiling point is a boiling point at 1 atm.

The high-boiling-point solvent, whose characteristic is a high boiling point, may have an excessively high boiling point at 1 atm, which causes the high-boiling-point solvent to decompose, leading to inability to measure an accurate boiling point. In such a case, the boiling point at a reduced pressure may be measured, and converted into a boiling point at 1 atm using a common boiling point conversion table.

Further, in the hafnium polyoxometalate compound-containing material of the present invention, components other than a component derived from hafnium or hafnium polyoxometalate, an organic nitrogen compound, and a component derived from hydrogen peroxide (referred to as "other components") may be contained as inevitable impurities as long as the effects of the material are not inhibited.

To the hafnium polyoxometalate compound-containing material of the present invention, a dispersant, a pH adjuster, a colorant, a thickener, a wetting agent, a binder resin and the like may be appropriately added according to a use purpose.

A method for producing a hafnium polyoxometalate compound-containing material of the present invention will be described below.

The method for producing a hafnium polyoxometalate compound-containing material according to the present invention includes the steps of: forming a hafnium-containing precipitate by adding an acidic hafnium aqueous solution containing hafnium to an alkaline aqueous solution; and forming a hafnium polyoxometalate compound-containing material by adding an organic nitrogen compound to a hafnium-containing precipitation slurry obtained by making the hafnium-containing precipitate into slurry.

First, hydrofluoric acid (HF) is added as a hydrofluoric acid aqueous solution or the like to hafnium, a hafnium oxide or hafnium hydroxide, and the mixture maintained at 60°C to 100°C in a water bath and held for 1 hour to 72 hours to react the mixture, thereby obtaining hafnium fluoride (HfF₄), which is dissolved in water to form a hydrofluoric acid solution of a hafnium compound.

Here, the hydrofluoric acid solution of a hafnium compound is preferably adjusted by adding water (for example, pure water) so that hafnium is contained at 1 to 100 g/L in terms of HfO₂. Here, the hafnium content is preferably 1 g/L or more in terms of HfO₂ because a hafnium polyoxometalate compound hydrate that is easily soluble in water is obtained. From the viewpoint of productivity, the hafnium content is more preferably 10 g/L or more, and still more preferably 20 g/L or more. On the other hand, the hafnium content is preferably 100 g/L or less in terms of HfO₂ because a hafnium polyoxometalate compound hydrate that is easily soluble in water is obtained. For synthesizing a hafnium polyoxometalate compound hydrate that is more reliably soluble in water, the hafnium content is more preferably 90 g/L or less, still more preferably 80 g/L or less, and particularly preferably 70 g/L or less. The pH of the hydrofluoric acid solution of a hafnium compound is preferably 2 or less, and more preferably 1 or less, from the viewpoint of completely dissolving hafnium or hafnium oxide.

Next, hydrogen peroxide is added to the hydrofluoric acid solution of a hafnium compound, and mixed to obtain an acidic or neutral hafnium complex aqueous solution. It is presumed that at least a part of hafnium contained in the obtained hafnium complex aqueous solution forms a peroxo complex.

Here, the hydrogen peroxide content of a hydrogen peroxide solution added to the hydrofluoric acid solution of a hafnium compound is preferably 0.5 mass% to 35 mass%. Hydrogen peroxide is added such that the molar ratio of hydrogen peroxide to hafnium, H₂O₂/Hf, is preferably 0.6 or more and 1.5 or less, and more preferably 0.7 or more and 1.2 or less because hydrogen peroxide may decompose during mixing.

In the step of forming a hafnium-containing precipitate by adding a hafnium complex aqueous solution to an alkaline aqueous solution, a precipitation slurry containing hafnium is obtained by addition of the hafnium complex aqueous solution to the alkaline aqueous solution, for example, aqueous ammonia, that is, a reverse neutralization method. Fluoride ions are removed from the obtained precipitation slurry containing hafnium to obtain a hafnium-containing precipitate from which fluoride ions have been removed.

The ammonia content of the aqueous ammonia used for reverse neutralization is preferably 10 mass% to 30 mass%. When the ammonia content is 10 mass%, hafnium hardly remains undissolved, and hafnium or hafnium polyoxometalate can be completely dissolved in water. On the other hand, the ammonia content is preferably 30 mass% or less because the aqueous ammonia is close to a saturated aqueous solution of ammonia.

From such a viewpoint, the ammonia content of aqueous ammonia is preferably 10 mass% or more, more preferably 15 mass% or more, still more preferably 20 mass% or more, and particularly preferably 25 mass% or more. On the other hand, the ammonia content is preferably 30 mass% or less, more preferably 29 mass% or less, and still more preferably 28 mass% or less.

For the amount of the hafnium complex aqueous solution added to the aqueous ammonia, in the reverse neutralization step, the molar ratio of NH₃/Hf is preferably 95 or more and 500 or less, more preferably 100 or more and 450 or less, and still more preferably 110 or more and 400 or less. For the amount of the hafnium complex aqueous solution added to the aqueous ammonia, the molar ratio of NH₃/HF is preferably 3.0 or more, more preferably 4.0 or more, and still more preferably 5.0 or more, from the viewpoint of forming a hafnium polyoxometalate compound that is soluble in amine or dilute aqueous ammonia. On the other hand, from the viewpoint of cost reduction, the molar ratio of NH₃/HF is preferably 100 or less, more preferably 50 or less, and still more preferably 40 or less.

In the reverse neutralization step, the time taken to add the hafnium complex aqueous solution to the aqueous ammonia is preferably 10 minutes or less, more preferably 8 minutes or less, and still more preferably 5 minutes or less. That is, it is preferable that instead of being slowly added over time, the hafnium complex aqueous solution is added to aqueous ammonia as quickly as possible, for example, at once, and subjected to a neutralization reaction. In the reverse neutralization step, it is possible to carry out the neutralization reaction while maintaining a high pH because the hafnium complex aqueous solution is added to aqueous ammonia which is alkaline. The hafnium complex aqueous solution and the aqueous ammonia can be used at ordinary temperature.

In the reverse neutralization step, fluoride ions are removed from the precipitation slurry containing hafnium and obtained by the reverse neutralization method, thereby obtaining a hafnium-containing precipitate from which fluoride ions have been removed. In the precipitation slurry containing hafnium and obtained by the reverse neutralization method, fluorine compounds such as ammonium fluoride are present as impurities, and are preferably removed.

The method for removing fluorine compounds is not limited. It is possible to utilize, for example, arbitrary, and for example, filtration-based methods using a membrane, such as reverse osmosis filtration using aqueous ammonia and pure water, ultrafiltration, and microfiltration, centrifugation, or other known methods. Removal of fluoride ions from the precipitation slurry containing hafnium does not require temperature adjustment, and can be performed at ordinary temperature.

Specifically, the precipitation slurry containing hafnium obtained by the reverse neutralization method is decanted using a centrifuge, and washed a plurality of times until the amount of released fluoride ions is 100 mg/L or less, thereby obtaining a hafnium-containing precipitate from which fluoride ions have been removed is obtained. By repeating the decantation and the washing a plurality of times, for example, three times, the added fluoride ions are removed, together with hydrogen peroxide.

The cleansing liquid used for removing fluoride ions is preferably aqueous ammonia. Specifically, aqueous ammonia with an ammonia content of 1 mass% or more and 35 mass% or less is preferable. Such aqueous ammonia ensures that ammonia is appropriately related to fluoride ions, and an undesired cost increase can be avoided.

In this way, the formed hafnium-containing precipitate from which fluoride ions have been removed is diluted with pure water or the like to obtain a hafnium-containing precipitation slurry from which fluoride ions have been removed. For the hafnium content of the hafnium-containing precipitation slurry, a part of the hafnium-containing precipitation slurry is collected, dried at 110°C for 24 hours, and then fired at 1,000°C for 4 hours to form HfO₂. The weight of HfO₂ thus formed is measured. From the weight, the hafnium content of the hafnium-containing precipitation slurry can be calculated.

A mixture obtained by mixing the hafnium-containing precipitation slurry from which fluoride ions have been removed, the organic nitrogen compound, and pure water is held with stirring at 5°C to 90°C for 0.1 hours to 48 hours to obtain the hafnium polyoxometalate compound-containing material of the present invention.

The organic nitrogen compound mixed with the hafnium-containing precipitation slurry is more preferably quaternary ammonium as described above.

From the viewpoint of solubility, the quaternary ammonium is mixed such that the content of the quaternary ammonium in the hafnium-containing precipitation slurry is preferably 40 mass% or less, and more preferably 20 mass% or less. From the same viewpoint, the mixing is performed such that the content of the quaternary ammonium compound in the hafnium-containing precipitation slurry is preferably 0.1 mass% or more, and more preferably 1 mass% or more, and may be 5 mass% or more or 10 mass% or more. The quaternary ammonium is more preferably one or more selected from TMAH, TEAH, choline and benzyltrimethylammonium hydroxy.

The quaternary ammonium mixed with the hafnium-containing precipitation slurry may be a mixture of two or more, instead of one, selected from TMAH, TEAH, choline and benzyltrimethylammonium hydroxy. Examples thereof include a mixture of two kinds of quaternary ammonium such as TMAH and TEAH, TMAH and choline, TMAH and benzyltrimethylammonium hydroxy, a mixture of three kinds of quaternary ammonium such as TMAH, TEAH and choline, and a mixture of four kinds of quaternary ammonium such as TMAH, TEAH, choline and benzyltrimethylammonium hydroxy.

Hafnium polyoxometalate compound powder of the present invention contains hafnium polyoxometalate particles contained in the hafnium polyoxometalate compound-containing material of the present invention.

The hafnium polyoxometalate compound powder of the present invention includes dried powder obtained by drying, for example, vacuum drying of the hafnium polyoxometalate compound-containing material of the present invention, and fired powder obtained by firing the obtained dried powder. The hafnium polyoxometalate compound powder of the present invention also includes hafnium polyoxometalate compound powder generated by vacuum drying or firing of the hafnium polyoxometalate compound-containing material of the present invention and differing in physical properties such as a crystal structure, and may have an amorphous structure, a single crystal structure, or a polycrystalline structure.

Next, a method for producing hafnium polyoxometalate compound powder according to the present invention includes the step of forming hafnium oxide powder by drying and/or firing the hafnium polyoxometalate compound-containing material of the present invention.

Specifically, in a method for producing dried powder of a hafnium polyoxometalate compound, among the hafnium polyoxometalate compound powders, the hafnium polyoxometalate compound-containing material obtained by the method for producing a hafnium polyoxometalate compound-containing material according to the present invention is placed in a stationary furnace, and subjected to drying, for example, vacuum drying, at a heating temperature of about 60°C to 200°C for 1 hour to 72 hours to evaporate moisture of the hafnium polyoxometalate compound-containing material of the present invention, thereby obtaining dried powder of a hafnium polyoxometalate compound containing hafnium polyoxometalate particles contained in the hafnium polyoxometalate compound-containing material of the present invention.

On the other hand, in the method for producing fired powder of a hafnium polyoxometalate compound, the hafnium polyoxometalate compound-containing material of the present invention is subjected to vacuum drying as described above, and the obtained dried powder of a hafnium polyoxometalate compound is placed in a stationary furnace, and fired at a firing temperature of 300°C or higher and 1,200°C or lower for a firing time of 1 hour or more and 72 hours or less in the atmosphere to obtain fired powder of a hafnium polyoxometalate compound.

A pulverized product of the dried powder and the fired powder of the hafnium polyoxometalate compound may be used as hafnium polyoxometalate compound powder. Undersize particles (finer particles) obtained by classifying the dried powder and the fired powder of a hafnium polyoxometalate compound with a sieve, whether they are pulverized or not, may be used as hafnium polyoxometalate compound powder. Oversize particles (coarser particles) may be pulverized again, classified, and used. It is also possible to combine pulverization and classification by using a vibrating sieve including iron balls coated with nylon or fluororesin, or the like, as a pulverization medium. By combining classification and pulverization as described above, excessively large-sized hafnium compound powder, if any, can be removed. Specifically, when classification is performed using a sieve, a sieve having a mesh size of 150 µm to 1,000 µm is preferably used. When the mesh size is 150 µm to 1,000 µm, the proportion of oversize particles is not so high that re-pulverization is not repeated, and hafnium polyoxometalate compound powder that needs to be re-pulverized is not classified as undersize particles.

By mixing the thus-obtained hafnium polyoxometalate compound powder with water or an organic solvent as a dispersion medium, and wet-pulverizing the mixture with a medium such as beads, a hafnium polyoxometalate compound powder-containing liquid can be obtained. Here, examples of the organic solvent used as a dispersion medium include alcohols, esters, ketones, aromatic hydrocarbons, aliphatic hydrocarbons, ethers, and mixed solvents thereof. Further, a binder such as a resin component may be added for improving the ability to form a hafnium polyoxometalate compound film using the hafnium polyoxometalate compound powder-containing liquid. Examples of the resin component used as a binder include acrylic resins, polyurethane, epoxy resins, polystyrene, polycarbonate, glycol-based resins, cellulose-based resins, mixed resins thereof, and copolymer resins.

The hafnium polyoxometalate compound film of the present invention contains hafnium polyoxometalate particles contained in the hafnium polyoxometalate compound-containing material of the present invention.

The hafnium polyoxometalate compound film of the present invention includes a dried film obtained by applying the hafnium polyoxometalate compound-containing material of the present invention, followed by drying, for example, vacuum drying, and a fired film obtained by firing the obtained dried film. The hafnium polyoxometalate compound film of the present invention also includes a hafnium polyoxometalate compound films generated by vacuum drying or firing of the hafnium polyoxometalate compound-containing material of the present invention and differing in physical properties such as a crystal structure, and may have an amorphous structure, a single crystal structure, or a polycrystalline structure.

Next, a method for producing a hafnium polyoxometalate compound film according to the present invention includes applying, and drying and/or firing the hafnium polyoxometalate compound-containing material of the present invention.

Specifically, a method for producing a hafnium polyoxometalate compound dried film of the present invention, among the hafnium polyoxometalate compound films of the present invention, includes an application step of applying the hafnium polyoxometalate compound-containing material of the present invention to a surface of a substrate, and a film drying step of preparing a dried film by drying the hafnium polyoxometalate compound-containing material applied to the surface of the substrate.

Specifically, the hafnium polyoxometalate compound-containing material obtained by the method for producing a hafnium polyoxometalate compound-containing material according to the present invention is dropped onto a surface of a substrate with a syringe while being filtered through, for example, a filter having a pore diameter of 2 µm as necessary, and is applied by spin coating (1,500rpm, 30 sec). Next, the hafnium polyoxometalate compound-containing material is dried at 110°C or higher and lower than 300°C for 30 minutes to 12 hours to form a hafnium polyoxometalate compound dried film on the surface of the substrate.

When the hafnium polyoxometalate compound-containing material obtained by the method for producing a hafnium polyoxometalate compound-containing material according to the present invention is a high-viscosity liquid or is gelled, the hafnium polyoxometalate compound-containing material may be applied onto the surface of the substrate using a brush or the like. Next, the hafnium polyoxometalate compound-containing material is dried at 110°C or higher and lower than 300°C for 30 minutes to 12 hours to form a hafnium polyoxometalate compound dried film on the surface of the substrate. It is also possible to form the hafnium polyoxometalate compound dried film according to the present invention on the surface of the substrate by performing the drying for a long time of 1 hour to 3 days at a temperature that is equal to or higher than room temperature and lower than 110°C.

Specifically, a method for producing a hafnium polyoxometalate compound fired film of the present invention, among the hafnium polyoxometalate compound films of the present invention, includes an application step of applying the hafnium polyoxometalate compound-containing material of the present invention to a surface of a substrate, a film drying step of preparing a dried film by drying the hafnium polyoxometalate compound-containing material applied to the surface of the substrate, in the atmosphere or under vacuum, and a film firing step of preparing a fired film by firing the dried film at a firing temperature of 300°C or higher and 1,200°C or lower for a firing time of 1 hour or more and 12 hours or less in the atmosphere.

Specifically, a substrate on which a hafnium polyoxometalate compound dried film obtained by applying the hafnium polyoxometalate compound-containing material of the present invention and performing drying as described above is placed in a stationary furnace, and fired at a firing temperature of 300°C or higher and 1,200°C or lower for a firing time of 1 hour or more and 12 hours or less in the atmosphere to form a hafnium polyoxometalate compound fired film according to the present invention on the surface of the substrate.

A composite hafnium polyoxometalate compound-containing material of the present invention includes the hafnium polyoxometalate compound-containing material of the present invention, and at least one element selected from the group consisting of Si, Al, Ti, Zn, Sn, Y, Ce, Ba, Sr, P, S, La, Gd, Nd, Eu, Dy, Yb, Nb, Li, Na, K, Mg, Ca, Zr, Mo, Ta, V, Ga, Ge and W.

It is presumed that the composite hafnium polyoxometalate compound-containing material of the present invention is present in the containing material as ions in which hafnium polyoxometalate and at least one element selected from the above-mentioned group are ionically bonded. Here, the composite hafnium polyoxometalate compound-containing material of the present invention is not limited to a "containing liquid" in the present invention, and includes the "containing liquid" in which a gel-like precipitate is generated. The hafnium polyoxometalate content in the composite hafnium polyoxometalate compound-containing material of the present invention can be calculated by diluting the composition with dilute hydrochloric acid as necessary, and measuring the Hf weight fraction in terms of HfO₂ in a manner conforming to JIS K0116:2014 by ICP emission spectrometry (AG-5110 manufactured by Agilent Technologies).

At least one element selected from the above-described group may be contained as a compound. Here, examples of the compound include oxides, metal acid alkali metal salts, metal acid alkaline earth metal salts, chlorides, metal acid alkoxides, and polyoxometalate. For the content of at least one element selected from the above-described group, the molar ratio of the total mole number content of each element in terms of metal (X) with respect to hafnium (Hf), X/Hf, where X is the total mole number content of each element in terms of metal, may be 0.001 to 75, 0.002 to 50, 0.01 to 40, 0.2 to 30, 0.5 to 25, 0.8 to 1.5, 0.8 to 1.3, 0.9 to 1.2, or 0.9 to 1.1. The content is calculated in terms of P when the element is P, and in terms of S when the element is S.

A method for producing the composite hafnium polyoxometalate compound-containing material of the present invention includes the step of forming a composite hafnium polyoxometalate compound material by mixing the hafnium polyoxometalate compound-containing material formed by the method for producing a hafnium polyoxometalate compound-containing material according to the present invention and at least one element selected from the group consisting of Si, Al, Ti, Zn, Sn, Y, Ce, Ba, Sr, P, S, La, Gd, Nd, Eu, Dy, Yb, Nb, Li, Na, K, Mg, Ca, Zr, Ta, V, Ga, Ge and W.

A mixture obtained by mixing the hafnium polyoxometalate compound-containing material of the present invention and at least one element selected from the above-mentioned group is stirred, with the liquid temperature maintained at an appropriate temperature for a predetermined time, thereby obtaining the composite hafnium polyoxometalate compound-containing material of the present invention. Here, the at least one element which is selected from the above-mentioned group and mixed with the hafnium polyoxometalate compound-containing material formed by the method for producing a hafnium polyoxometalate compound-containing material according to the present invention may be in the form of any of polyoxometalate, an oxide including a peroxo complex, a hydroxide, a metal complex, a salt and the like.

For example, a method for producing a tantalum polyoxometalate compound-containing liquid, which is one of the elements mixed with the hafnium polyoxometalate compound-containing material of the present invention, will be described below.

First, tantalum, tantalum oxide, tantalum hydroxide or tantalum alkoxide is reacted with hydrofluoric acid (HF) such as an aqueous hydrofluoric acid solution to obtain tantalum fluoride (H₂TaF₇), which is dissolved in water to obtain a tantalum fluoride aqueous solution which is an acidic metal aqueous solution. In the case of tantalum chloride, the step of dissolution in hydrofluoric acid is omitted, and water is added to tantalum chloride, whereby an acidic tantalum aqueous solution can be formed.

Here, the tantalum fluoride aqueous solution is preferably adjusted by adding water (for example, pure water) so that tantalum is contained at 1 to 100 g/L in terms of Ta₂O₅. Here, the tantalum content is preferably 1 g/L or more in terms of Ta₂O₅ because a tantalum polyoxometalate compound hydrate that is easily soluble in water is obtained. From the viewpoint of productivity, the hafnium content is more preferably 10 g/L or more, and still more preferably 20 g/L or more. On the other hand, the tantalum content is preferably 100 g/L or less in terms of Ta₂O₅ because a tantalum polyoxometalate compound hydrate that is easily soluble in water is obtained. For synthesizing a tantalum polyoxometalate compound hydrate that is more reliably soluble in water, the tantalum content is more preferably 90 g/L or less, still more preferably 80 g/L or less, and particularly preferably 70 g/L or less. The pH of the tantalum fluoride aqueous solution is preferably 2 or less, and more preferably 1 or less, from the viewpoint of completely dissolving tantalum or tantalum oxide.

Next, the tantalum fluoride aqueous solution is subjected to a neutralization reaction by use of an alkaline aqueous solution to obtain a fluorine-containing tantalum hydrate cake. Here, the alkaline aqueous solution used for neutralizing the tantalum fluoride aqueous solution is preferably aqueous ammonia with an ammonia content of 10 mass% to 30 mass%.

The ammonia content of the aqueous ammonia used for neutralization is preferably 10 mass% to 30 mass%. When the ammonia content is 10 mass%, tantalum hardly remains undissolved, and tantalum or tantalum polyoxometalate can be completely dissolved in water. On the other hand, the ammonia content is preferably 30 mass% or less because the aqueous ammonia is close to a saturated aqueous solution of ammonia.

From such a viewpoint, the ammonia content of aqueous ammonia is preferably 10 mass% or more, more preferably 15 mass% or more, still more preferably 20 mass% or more, and particularly preferably 25 mass% or more. On the other hand, the ammonia content is preferably 30 mass% or less, more preferably 29 mass% or less, and still more preferably 28 mass% or less.

For the addition amount, in the neutralization step, the molar ratio of NH₃/Ta is preferably 95 or more and 500 or less, more preferably 100 or more and 450 or less, and still more preferably 110 or more and 400 or less. For the addition amount, the molar ratio of NH₃/HF is preferably 3.0 or more, more preferably 4.0 or more, and still more preferably 5.0 or more, from the viewpoint of forming a tantalum polyoxometalate compound that is soluble in amine or dilute aqueous ammonia. On the other hand, from the viewpoint of cost reduction, the molar ratio of NH₃/HF is preferably 100 or less, more preferably 50 or less, and still more preferably 40 or less.

The addition time in the neutralization reaction is preferably 10 minutes or less, more preferably 8 minutes or less, and still more preferably 5 minutes or less. That is, it is preferable that instead of being slowly added over time, the reactant is added as quickly as possible, for example, at once, and subjected to a neutralization reaction. The tantalum fluoride aqueous solution and the aqueous ammonia can be used at ordinary temperature.

The fluorine-containing tantalum hydrate cake obtained by the neutralization reaction is decanted with diluted aqueous ammonia using a centrifuge, and repeatedly washed until the amount of released fluoride ions is 100 mg/L or less, whereby fluoride ions are removed from the fluorine-containing tantalum hydrate cake to obtain a tantalum-containing precipitate. In the fluorine-containing tantalum hydrate cake obtained by the neutralization reaction, fluorine compounds such as ammonium fluoride are present as impurities, and are preferably removed.

For the tantalum content of the tantalum-containing precipitate obtained, a part of the tantalum-containing precipitate is collected, dried at 110°C for 24 hours, and then fired at 1,000°C for 4 hours to form Ta₂O₅. The weight of Ta₂O₅ thus formed is measured. From the weight, the tantalum content of the tantalum-containing precipitate can be calculated.

The cleansing liquid used for removing fluoride ions is preferably diluted aqueous ammonia. Specifically, diluted aqueous ammonia with an ammonia content of 1 mass% or more and 35 mass% or less is preferable. Such diluted aqueous ammonia ensures that ammonia and ammonium ions are appropriately related to fluorine, and an undesired cost increase can be avoided.

The method for removing fluorine compounds is not limited. It is possible to utilize, for example, arbitrary, and for example, filtration-based methods using a membrane, such as reverse osmosis filtration using aqueous ammonia and pure water, ultrafiltration, and microfiltration, centrifugation, or other known methods. Removal of fluoride ions from the fluorine-containing tantalum hydrate cake does not require temperature adjustment, and can be performed at ordinary temperature.

To the tantalum-containing precipitate obtained, a tertiary amine compound and pure water are added, and the mixture is stirred for 10 minutes to obtain a tantalum-containing mixed liquid. Thereafter, 35 mass% hydrogen peroxide is added to the tantalum-containing mixed liquid, and the mixture is stirred for 30 minutes to obtain a tantalum polyoxometalate compound-containing liquid.

From the viewpoint of solubility, the tertiary amine compound is mixed such that the content of the tertiary amine compound in the tantalum-containing mixed liquid is preferably 30 mass% or less, and more preferably 20 mass% or less. From the same viewpoint, the mixing is performed such that the content of the tertiary amine compound in the tantalum-containing mixed liquid is preferably 0.1 mass% or more, and more preferably 1 mass% or more, and may be 5 mass% or more or 10 mass% or more. Further, the tertiary amine compound is preferably one or more selected from trimethylamine, triethylamine and tri-n-propylamine.

The hydrogen peroxide content of the hydrogen peroxide solution is preferably 0.5 mass% to 35 mass%. Hydrogen peroxide is added such that the molar ratio of hydrogen peroxide to tantalum, H₂O₂/Ta, is preferably 0.6 or more and 1.5 or less, and more preferably 0.7 or more and 1.2 or less because hydrogen peroxide may decompose during mixing.

The method for producing a tantalum polyoxometalate compound-containing liquid preferably includes the step of removing hydrogen peroxide from the obtained tantalum polyoxometalate compound-containing liquid. This is because if the tantalum polyoxometalate compound-containing liquid contains hydrogen peroxide, there may be a risk that the inside of a closed container is filled with gas produced by decomposition of hydrogen peroxide, so that the container expands, or ruptures in the worst case.

The method for removing hydrogen peroxide is not limited, and examples thereof include stirring under open and reduced-pressure conditions, and drying under reduced pressure.

A method for producing a niobium polyoxometalate compound-containing liquid, which is one of the elements mixed with the hafnium polyoxometalate compound-containing material of the present invention, will be described below. The description of parts overlapping the method for producing the tantalum polyoxometalate compound-containing liquid is omitted.

First, niobium, niobium oxide or niobium hydroxide is reacted with hydrofluoric acid (HF) such as an aqueous hydrofluoric acid solution to obtain niobium fluoride (H₂NbF₇), which is dissolved in water to obtain a niobium fluoride aqueous solution which is an acidic metal aqueous solution. In the case of niobium chloride, the step of dissolution in hydrofluoric acid is omitted, and water is added to niobium chloride, whereby an acidic niobium aqueous solution can be formed.

Here, the niobium fluoride aqueous solution is preferably adjusted by adding water (for example, pure water) so that niobium is contained at 1 to 100 g/L in terms of Nb₂O₅. Here, the niobium content is preferably 1 g/L or more in terms of Nb₂O₅ because a niobium polyoxometalate compound hydrate that is easily soluble in water is obtained. From the viewpoint of productivity, the hafnium content is more preferably 10 g/L or more, and still more preferably 20 g/L or more. On the other hand, the niobium content is preferably 100 g/L or less in terms of Nb₂O₅ because a niobium polyoxometalate compound hydrate that is easily soluble in water is obtained. For synthesizing a niobium polyoxometalate compound hydrate that is more reliably soluble in water, the niobium content is more preferably 90 g/L or less, still more preferably 80 g/L or less, and particularly preferably 70 g/L or less. The pH of the niobium fluoride aqueous solution is preferably 2 or less, and more preferably 1 or less, from the viewpoint of completely dissolving niobium or niobium oxide.

Next, the niobium fluoride aqueous solution is subjected to a neutralization reaction by use of an alkaline aqueous solution to obtain a fluorine-containing niobium hydrate cake. Here, the alkaline aqueous solution used for neutralizing the niobium fluoride aqueous solution is preferably aqueous ammonia with an ammonia content of 10 mass% to 30 mass%.

For the addition amount, in the neutralization step, the molar ratio of NH₃/Nb is preferably 95 or more and 500 or less, more preferably 100 or more and 450 or less, and still more preferably 110 or more and 400 or less. For the addition amount, the molar ratio of NH₃/HF is preferably 3.0 or more, more preferably 4.0 or more, and still more preferably 5.0 or more, from the viewpoint of forming a niobium polyoxometalate compound that is soluble in amine or dilute aqueous ammonia. On the other hand, from the viewpoint of cost reduction, the molar ratio of NH₃/HF is preferably 100 or less, more preferably 50 or less, and still more preferably 40 or less.

The addition time in the neutralization reaction is preferably 10 minutes or less, more preferably 8 minutes or less, and still more preferably 5 minutes or less.

The fluorine-containing niobium hydrate cake obtained by the neutralization reaction is decanted with diluted aqueous ammonia using a centrifuge, and repeatedly washed until the amount of released fluoride ions is 100 mg/L or less, whereby fluoride ions are removed from the fluorine-containing niobium hydrate cake to obtain a niobium-containing precipitate. In the fluorine-containing niobium hydrate cake obtained by the neutralization reaction, fluorine compounds such as ammonium fluoride are present as impurities, and are preferably removed. The cleansing liquid used for removing fluoride ions is preferably diluted aqueous ammonia.

For the niobium content of the niobium-containing precipitate obtained, a part of the niobium-containing precipitate is collected, dried at 110°C for 24 hours, and then fired at 1,000°C for 4 hours to form Nb₂O₅. The weight of Nb₂O₅ thus formed is measured. From the weight, the niobium content of the niobium-containing precipitate can be calculated.

To the niobium-containing precipitate obtained, a tertiary amine compound and pure water are added, and the mixture is stirred for 10 minutes to obtain a niobium-containing mixed liquid. Thereafter, 35 mass% hydrogen peroxide is added to the niobium-containing mixed liquid, and the mixture is stirred for 30 minutes to obtain a niobium polyoxometalate compound-containing liquid.

The tertiary amine compound is mixed such that the content of the tertiary amine compound in the tantalum-containing mixed liquid is preferably 0.1 mass% or more and 30 mass% or less. The tertiary amine compound is preferably one or more selected from trimethylamine, triethylamine and tri-n-propylamine.

The hydrogen peroxide content of the hydrogen peroxide solution is preferably 0.5 mass% to 35 mass%. Hydrogen peroxide is added such that the molar ratio of hydrogen peroxide to niobium, H₂O₂/Nb, is preferably 0.6 or more and 1.5 or less, and more preferably 0.7 or more and 1.2 or less because hydrogen peroxide may decompose during mixing.

Like the method for producing a tantalum polyoxometalate compound-containing liquid, the method for producing a niobium polyoxometalate compound-containing liquid preferably includes the step of removing hydrogen peroxide from the obtained niobium polyoxometalate compound-containing liquid.

A method for producing a titanium polyoxometalate compound-containing liquid, which is one of the elements mixed with the hafnium polyoxometalate compound-containing material of the present invention, will be described below. The description of parts overlapping the method for producing the tantalum polyoxometalate compound-containing liquid is omitted.

First, titanium, titanium oxide or titanium hydroxide is reacted with hydrofluoric acid (HF) such as an aqueous hydrofluoric acid solution to obtain titanium fluoride (H₂TiF₆), which is dissolved in water to obtain a titanium fluoride aqueous solution which is an acidic metal aqueous solution. In the case of titanium chloride or titanyl sulfate, the step of dissolution in hydrofluoric acid is omitted, and water is added to the compound, whereby an acidic titanium aqueous solution can be formed.

Here, the titanium fluoride aqueous solution is preferably adjusted by adding water (for example, pure water) so that titanium is contained at 1 to 100 g/L in terms of TiO₂. Here, the titanium content is preferably 1 g/L or more in terms of TiO₂ because a titanium polyoxometalate compound hydrate that is easily soluble in water is obtained. From the viewpoint of productivity, the hafnium content is more preferably 10 g/L or more, and still more preferably 20 g/L or more. On the other hand, the titanium content is preferably 100 g/L or less in terms of TiO₂ because a titanium polyoxometalate compound hydrate that is easily soluble in water is obtained. For synthesizing a titanium polyoxometalate compound hydrate that is more reliably soluble in water, the titanium content is more preferably 90 g/L or less, still more preferably 80 g/L or less, and particularly preferably 70 g/L or less. The pH of the titanium fluoride aqueous solution is preferably 2 or less, and more preferably 1 or less, from the viewpoint of completely dissolving titanium or titanium oxide.

Next, the titanium fluoride aqueous solution is subjected to a neutralization reaction by use of an alkaline aqueous solution to obtain a fluorine-containing titanium hydrate cake. Here, the alkaline aqueous solution used for neutralizing the titanium fluoride aqueous solution is preferably aqueous ammonia with an ammonia content of 10 mass% to 30 mass%.

For the addition amount, in the neutralization step, the molar ratio of NH₃/Ti is preferably 95 or more and 500 or less, more preferably 100 or more and 450 or less, and still more preferably 110 or more and 400 or less. For the addition amount, the molar ratio of NH₃/HF is preferably 3.0 or more, more preferably 4.0 or more, and still more preferably 5.0 or more, from the viewpoint of forming a titanium polyoxometalate compound that is soluble in amine or dilute aqueous ammonia. On the other hand, from the viewpoint of cost reduction, the molar ratio of NH₃/HF is preferably 100 or less, more preferably 50 or less, and still more preferably 40 or less.

The addition time in the neutralization reaction is preferably 10 minutes or less, more preferably 8 minutes or less, and still more preferably 5 minutes or less.

The fluorine-containing titanium hydrate cake obtained by the neutralization reaction is decanted with diluted aqueous ammonia using a centrifuge, and repeatedly washed until the amount of released fluoride ions is 100 mg/L or less, whereby fluoride ions are removed from the fluorine-containing titanium hydrate cake to obtain a titanium-containing precipitate. In the fluorine-containing titanium hydrate cake obtained by the neutralization reaction, fluorine compounds such as ammonium fluoride are present as impurities, and are preferably removed. The cleansing liquid used for removing fluoride ions is preferably diluted aqueous ammonia.

For the niobium content of the titanium-containing precipitate obtained, a part of the titanium-containing precipitate is collected, dried at 110°C for 24 hours, and then fired at 1,000°C for 4 hours to form TiO₂. The weight of TiO₂ thus formed is measured. From the weight, the titanium content of the titanium-containing precipitate can be calculated.

To the titanium-containing precipitate obtained, a tertiary amine compound and pure water are added, and the mixture is stirred for 10 minutes to obtain a titanium-containing mixed liquid. Thereafter, 35 mass% hydrogen peroxide is added to the titanium-containing mixed liquid, and the mixture is stirred for 30 minutes to obtain a titanium polyoxometalate compound-containing liquid.

The tertiary amine compound is mixed such that the content of the tertiary amine compound in the titanium-containing mixed liquid is preferably 0.1 mass% or more and 30 mass% or less. The tertiary amine compound is preferably one or more selected from trimethylamine, triethylamine and tri-n-propylamine.

The hydrogen peroxide content of the hydrogen peroxide solution is preferably 0.5 mass% to 35 mass%. Hydrogen peroxide is added such that the molar ratio of hydrogen peroxide to titanium, H₂O₂/Ti, is preferably 0.6 or more and 1.5 or less, and more preferably 0.7 or more and 1.2 or less because hydrogen peroxide may decompose during mixing.

Like the method for producing a tantalum polyoxometalate compound-containing liquid, the method for producing a titanium polyoxometalate compound-containing liquid preferably includes the step of removing hydrogen peroxide from the obtained titanium polyoxometalate compound-containing liquid.

A method for producing a zirconium polyoxometalate compound-containing liquid, which is one of the elements mixed with the hafnium polyoxometalate compound-containing material of the present invention, will be described below. The description of parts overlapping the method for producing the tantalum polyoxometalate compound-containing liquid is omitted.

First, zirconium, zirconium oxide or titanium hydroxide is reacted with hydrofluoric acid (HF) such as an aqueous hydrofluoric acid solution to obtain zirconium fluoride (H₂ZrF₆), which is dissolved in water to obtain a zirconium fluoride aqueous solution which is an acidic metal aqueous solution. In the case of zirconium chloride or zirconium sulfate, the step of dissolution in hydrofluoric acid is omitted, and water is added to the compound, whereby an acidic titanium aqueous solution can be formed.

Here, the zirconium fluoride aqueous solution is preferably adjusted by adding water (for example, pure water) so that zirconium is contained at 1 to 100 g/L in terms of ZrO₂. Here, the zirconium content is preferably 1 g/L or more in terms of ZrO₂ because a zirconium polyoxometalate compound hydrate that is easily soluble in water is obtained. From the viewpoint of productivity, the hafnium content is more preferably 10 g/L or more, and still more preferably 20 g/L or more. On the other hand, the zirconium content is preferably 100 g/L or less in terms of ZrO₂ because a zirconium polyoxometalate compound hydrate that is easily soluble in water is obtained. For synthesizing a zirconium polyoxometalate compound hydrate that is more reliably soluble in water, the zirconium content is more preferably 90 g/L or less, still more preferably 80 g/L or less, and particularly preferably 70 g/L or less. The pH of the zirconium fluoride aqueous solution is preferably 2 or less, and more preferably 1 or less, from the viewpoint of completely dissolving zirconium or zirconium oxide.

Next, the zirconium fluoride aqueous solution is subjected to a neutralization reaction by use of an alkaline aqueous solution to obtain a fluorine-containing zirconium hydrate cake. Here, the alkaline aqueous solution used for neutralizing the zirconium fluoride aqueous solution is preferably aqueous ammonia with an ammonia content of 10 mass% to 30 mass%.

For the addition amount, in the neutralization step, the molar ratio of NH₃/Zr is preferably 95 or more and 500 or less, more preferably 100 or more and 450 or less, and still more preferably 110 or more and 400 or less. For the addition amount, the molar ratio of NH₃/HF is preferably 3.0 or more, more preferably 4.0 or more, and still more preferably 5.0 or more, from the viewpoint of forming a zirconium polyoxometalate compound that is soluble in amine or dilute aqueous ammonia. On the other hand, from the viewpoint of cost reduction, the molar ratio of NH₃/HF is preferably 100 or less, more preferably 50 or less, and still more preferably 40 or less.

The addition time in the neutralization reaction is preferably 10 minutes or less, more preferably 8 minutes or less, and still more preferably 5 minutes or less.

The fluorine-containing zirconium hydrate cake obtained by the neutralization reaction is decanted with diluted aqueous ammonia using a centrifuge, and repeatedly washed until the amount of released fluoride ions is 100 mg/L or less, whereby fluoride ions are removed from the fluorine-containing zirconium hydrate cake to obtain a zirconium-containing precipitate. In the fluorine-containing zirconium hydrate cake obtained by the neutralization reaction, fluorine compounds such as ammonium fluoride are present as impurities, and are preferably removed. The cleansing liquid used for removing fluoride ions is preferably diluted aqueous ammonia.

For the zirconium content of the zirconium-containing precipitate obtained, a part of the titanium-containing precipitate is collected, dried at 110°C for 24 hours, and then fired at 1,000°C for 4 hours to form ZrO₂. The weight of ZrO₂ thus formed is measured. From the weight, the zirconium content of the zirconium-containing precipitate can be calculated.

To the zirconium-containing precipitate obtained, a tertiary amine compound and pure water are added, and the mixture is stirred for 10 minutes to obtain a zirconium-containing mixed liquid. Thereafter, 35 mass% hydrogen peroxide is added to the zirconium-containing mixed liquid, and the mixture is stirred for 30 minutes to obtain a zirconium polyoxometalate compound-containing liquid.

The tertiary amine compound is mixed such that the content of the tertiary amine compound in the zirconium-containing mixed liquid is preferably 0.1 mass% or more and 30 mass% or less. The tertiary amine compound is preferably one or more selected from trimethylamine, triethylamine and tri-n-propylamine.

The hydrogen peroxide content of the hydrogen peroxide solution is preferably 0.5 mass% to 35 mass%. Hydrogen peroxide is added such that the molar ratio of hydrogen peroxide to zirconium, H₂O₂/Zr, is preferably 0.6 or more and 1.5 or less, and more preferably 0.7 or more and 1.2 or less because hydrogen peroxide may decompose during mixing.

Like the method for producing a tantalum polyoxometalate compound-containing liquid, the method for producing a zirconium polyoxometalate compound-containing liquid preferably includes the step of removing hydrogen peroxide from the obtained zirconium polyoxometalate compound-containing liquid.

A method for producing a silicon polyoxometalate compound-containing liquid, which is one of the elements mixed with the hafnium polyoxometalate compound-containing material of the present invention, will be described below. The description of parts overlapping the method for producing the tantalum polyoxometalate compound-containing liquid is omitted.

The method for producing a silicon polyoxometalate compound-containing liquid includes a mixing step of preparing a mixed liquid containing a precursor of a silicon compound by adding an acidic aqueous solution to a raw material substance containing silicon, stirring the mixture at 15°C or higher and 50°C or lower, and a stirring step of forming a silicon compound-containing liquid by adding a solution containing an organic nitrogen compound to the mixed liquid, and stirring the mixture at 15°C or higher and 50°C or lower.

First, in the mixing step, an acidic aqueous solution is added to a raw material substance containing silicon, and the mixture is stirred at 15°C or higher and 50°C or lower to obtain a mixed liquid containing a precursor of a silicon compound.

Examples of the raw material substance containing silicon include tetraethoxysilane (TEOS), tetramethoxysilane (TMOS), and sodium silicate (sodium silicate). In the raw material substance containing silicon, the raw material substance containing silicon preferably contains at least one of tetraethoxysilane and sodium silicate.

Examples of the acidic aqueous solution include acetic acid, hydrochloric acid, sulfuric acid, and phosphoric acid. The acidic aqueous solution preferably contains at least one of acetic acid, hydrochloric acid and phosphoric acid.

When the acidic aqueous solution includes acetic acid, the acetic acid content is preferably 0.001 mass% to 3.0 mass%. When the acetic acid content is 0.001 mass% or more, in the case where the raw material substance containing silicon is TEOS, the silanol ester group Si-O-CH₂-OH₃ in TEOS can be efficiently hydrolyzed to form a Si-OH structure. On the other hand, the acetic acid content is preferably 3.0 mass% or less because the content of acetic acid contained in the silicon polyoxometalate compound-containing liquid, which is a final product, can be reduced. From such a viewpoint, the acetic acid content is preferably 0.001 mass% or more, more preferably 0.005 mass% or more, and still more preferably 0.01 mass% or more. On the other hand, the acetic acid content is preferably 3.0 mass% or less, more preferably 2.5 mass% or less, still more preferably 2.0 mass% or less, particularly preferably 1.5 mass% or less, more particularly preferably 1.0 mass% or less, still more particularly preferably 0.5 mass% or less, even more particularly preferably 0.1 mass% or less, especially preferably 0.05 mass% or less, more especially preferably 0.04 mass% or less, still more especially 0.03 mass% or less, and even more especially preferably 0.02 mass% or less. Unless otherwise specified, the acetic acid content described in the present specification is a content at the time of addition to the raw material substance containing silicon in the mixing step.

The acetic acid content in the silicon polyoxometalate compound-containing liquid is preferably 0.02 mass% to 10 mass%. The acetic acid content is more preferably 0.05 mass% or more, still more preferably 0.1 mass% or more, particularly preferably 0.15 mass% or more, and more particularly preferably 0.2 mass% or more. On the other hand, the acetic acid content is more preferably 5% by mass or less, still more preferably 3% by mass or less, particularly preferably 1% by mass or less, and particularly preferably 0.5% by mass or less.

Further, for the amount of acetic acid added to the raw material substance containing silicon, the molar ratio of CH₃COOH/Si is preferably 0.01 or more and 0.3 or less, more preferably 0.02 or more and 0.25 or less, and still more preferably 0.03 or more and 0.2 or less. Further, the time taken to add acetic acid to the raw material substance containing silicon is preferably 5 minutes or less, more preferably 3 minutes or less, and still more preferably 1 minute or less. For the acetic acid content in the silicon polyoxometalate compound-containing liquid, the molar ratio of CH₃COOH/Si is preferably 0.01 or more and 0.3 or less, more preferably 0.02 or more and 0.25 or less, and still more preferably 0.03 or more and 0.2 or less.

When the acidic aqueous solution added to the raw material substance containing silicon includes hydrochloric acid, the hydrochloric acid content is preferably 0.001 mass% to 3.0 mass%. When the hydrochloric acid content is 0.001 mass% or more, in the case where the raw material substance containing silicon is sodium silicate, sodium silicate can be efficiently formed from the sodium silicate. On the other hand, the hydrochloric acid content is preferably 3.0 mass% or less because hydrochloric acid hardly remains in the silicon polyoxometalate compound-containing liquid which is a final product. From such a viewpoint, the hydrochloric acid content is preferably 0.001 mass% or more, more preferably 0.005 mass% or more, and still more preferably 0.01 mass% or more. On the other hand, the hydrochloric acid content is preferably 3.0 mass% or less, more preferably 2.5 mass% or less, still more preferably 2.0 mass% or less, particularly preferably 1.5 mass% or less, more particularly preferably 1.0 mass% or less, still more particularly preferably 0.5 mass% or less, even more particularly preferably 0.1 mass% or less, especially preferably 0.05 mass% or less, more especially preferably 0.04 mass% or less, still more especially 0.03 mass% or less, and even more especially preferably 0.02 mass% or less. Unless otherwise specified, the hydrochloric acid content described in the present specification is a content at the time of addition to the raw material substance containing silicon in the mixing step. The value of the content is calculated in terms of weight of HCl in a 0.5 N hydrochloric acid aqueous solution.

The hydrochloric acid content in the silicon polyoxometalate compound-containing liquid is preferably 0.02 mass% to 10 mass%. The hydrochloric acid content is more preferably 0.05 mass% or more, still more preferably 0.1 mass% or more, particularly preferably 0.15 mass% or more, and more particularly preferably 0.2 mass% or more. On the other hand, the hydrochloric acid content is more preferably 5% by mass or less, still more preferably 3% by mass or less, particularly preferably 1% by mass or less, and particularly preferably 0.5% by mass or less.

Further, for the amount of hydrochloric acid added to the raw material substance containing silicon, the molar ratio of HCl/Si is preferably 0.5 or more and 1.5 or less, more preferably 0.55 or more and 1.3 or less, and still more preferably 0.6 or more and 1.1 or less. Further, the time taken to add hydrochloric acid to the raw material substance containing silicon is preferably 10 minutes or less, more preferably 5 minutes or less, and still more preferably 3 minutes or less. Further, for the hydrochloric acid content in the silicon polyoxometalate compound-containing liquid, the molar ratio of HCl/Si is preferably 0.5 or more and 1.5 or less, more preferably 0.55 or more and 1.3 or less, and still more preferably 0.6 or more and 1.1 or less.

In the mixing step, the liquid temperature of the mixed liquid in stirring after the acidic aqueous solution is added to the raw material substance containing silicon is preferably 15°C or higher and 50°C or lower, more preferably 20°C or higher and 45°C or lower, and still more preferably 25°C or higher and 40°C or lower. If the liquid temperature of the mixed liquid exceeds 50°C, moisture in the mixed liquid is so heavily evaporated that dehydration-condensation of Si-OH occurs in the mixed liquid, which causes partial formation of SiO₂ fine particles, leading to a possibility that at the time of adding the solution containing an organic nitrogen compound in the stirring step, dissolution does not occur.

Further, in the mixing step, the time taken for stirring after the acidic aqueous solution is added to the raw material substance containing silicon varies depending on a type of the raw material containing silicon, an amount of the acidic aqueous solution added, reduced-pressure conditions, and the like.

Specifically, when the raw material substance containing silicon is TEOS and the acidic aqueous solution includes acetic acid, the time taken for stirring is preferably 30 minutes or more and 24 hours or less, more preferably 1 hour or more and 15 hours or less, and still more preferably 2 hours or more and 12 hours or less.

When the raw material substance containing silicon is sodium silicate and the acidic aqueous solution includes hydrochloric acid, the time taken for stirring is preferably 10 minutes or more and 10 hours or less, more preferably 30 minutes or more and 7 hours or less, and still more preferably 1 hour or more and 5 hours or less.

In this way, an acidic aqueous solution is added to a raw material substance containing silicon, and the mixture is stirred at 15°C or higher and 50°C or lower to obtain a mixed liquid containing a precursor of a silicon compound.

It is presumed that in the precursor of a silicon compound, many of structures of silicic acid formed are linear due to addition of an acidic aqueous solution. This is presumed from the increased maximum intensity of absorption attributed to Si-OH stretching vibration in an infrared spectroscopic spectrum measured by a Fourier transform infrared spectrophotometer (FT-IR).

Further, when the raw material substance containing silicon is tetraethoxysilane and the acidic aqueous solution includes acetic acid, the process proceeds to the subsequent stirring step with respect to the obtained mixed liquid containing the precursor of a silicon compound.

On the other hand, when the raw material substance containing silicon is sodium silicate and the acidic aqueous solution includes hydrochloric acid, the following treatment is necessary as pretreatment for the process to proceed to the subsequent stirring step. The precursor of a silicon compound contains NaCl formed by reaction of Na of a part of sodium silicate and Cl of a part of hydrochloric acid during the stirring, and the NaCl is preferably removed from the precursor of a silicon compound. As a method for removing the NaCl, first, a mixed liquid containing the precursor of a silicon compound is placed in a centrifuge tube and centrifuged (4500rpm, 10 min) to precipitate a transparent gel, which is collected. Next, water is added to the collected transparent gel, the mixture is placed in a centrifuge tube, and centrifugation (4500 rpm, 20 min) is performed a plurality of times while the water is changed every time. In this way, NaCl can be removed from the precursor of a silicon compound.

Next, in the mixing step, a solution containing an organic nitrogen compound is added to the mixed liquid containing the precursor of a silicon compound, and the mixture is stirred at 15°C or higher and 50°C or lower to form a silicon polyoxometalate compound-containing liquid.

Examples of the organic nitrogen compound include primary amines, secondary amines, tertiary amines, and quaternary ammonium salts. The organic nitrogen compound preferably contains at least one of a primary amine, a secondary amine and a quaternary ammonium salt, and specific examples thereof include methylamine, ethylamine, dimethylamine, diethylamine, and tetramethylammonium hydroxide. Instead of the organic nitrogen compound, a solution containing an organic acid may be added to the mixed liquid containing the precursor of a silicon compound.

For the amount of the organic nitrogen compound added to the mixed liquid containing the precursor of a silicon compound, the molar ratio of amine/Si is preferably 0.5 or more and 15 or less, more preferably 1 or more and 10 or less, and still more preferably 1.5 or more and 5 or less. For the amount of the organic nitrogen compound added to the mixed liquid containing the precursor of a silicon compound, the molar ratio of amine/Si is preferably 1.7 or more, more preferably 1.3 or more, and still more preferably 1.3 or more, from the viewpoint of forming a silicon compound that is soluble in the organic nitrogen compound. On the other hand, from the viewpoint of cost reduction, the molar ratio of amine/Si is preferably 1.5 or less, more preferably 1.2 or less, and still more preferably 1 or less.

The time taken to add the organic nitrogen compound to the mixed liquid containing the precursor of a silicon compound is preferably 60 minutes or less, more preferably 30 minutes or less, and still more preferably 10 minutes or less.

In the stirring step, the liquid temperature in stirring after the solution containing the organic nitrogen compound is added to the mixed liquid containing the precursor of a silicon compound is preferably 15°C or higher and 50°C or lower, more preferably 20°C or higher and 45°C or lower, and still more preferably 25°C or higher and 40°C or lower. Here, if the liquid temperature in stirring exceeds 50°C, the organic nitrogen compound and the like are evaporated, so that the precursor of a silicon compound is hardly dissolved, leading to the increased possibility that the precursor of a silicon compound remains.

In the stirring step, the time taken for stirring after the solution containing the organic nitrogen compound is added to the mixed liquid containing the precursor of a silicon compound is preferably 10 minutes or more and 24 hours or less, more preferably 30 minutes or more and 20 hours or less, and still more preferably 1 hour or more and 15 hours or less.

By adding a solution containing an organic nitrogen compound to a mixed liquid containing a precursor of a silicon compound and stirring the mixture at 15°C or higher and 50°C or lower as described above, a silicon polyoxometalate compound-containing liquid can be formed. It is presumed that Si acid is easily made soluble in water because the precursor of a silicon compound is abundant in Si-OH structures.

A method for producing a molybdenum polyoxometalate compound-containing liquid, which is one of the elements mixed with the hafnium polyoxometalate compound-containing material of the present invention, will be described below. The description of parts overlapping the method for producing the tantalum polyoxometalate compound-containing liquid is omitted.

First, molybdenum or molybdenum oxide is reacted with hydrofluoric acid (HF) such as an aqueous hydrofluoric acid solution to obtain molybdenum fluoride (H₂MoF₆), which is dissolved in water to obtain a molybdenum fluoride aqueous solution which is an acidic metal aqueous solution.

Here, the molybdenum fluoride aqueous solution is preferably adjusted by adding water (for example, pure water) so that molybdenum is contained at 1 to 100 g/L in terms of MoO₃. Here, the molybdenum content is preferably 1 g/L or more in terms of MoO₃ because a molybdenum polyoxometalate compound hydrate that is easily soluble in water is obtained. From the viewpoint of productivity, the hafnium content is more preferably 10 g/L or more, and still more preferably 20 g/L or more. On the other hand, the molybdenum content is preferably 100 g/L or less in terms of MoO₃ because a molybdenum polyoxometalate compound hydrate that is easily soluble in water is obtained. For synthesizing a molybdenum polyoxometalate compound hydrate that is more reliably soluble in water, the molybdenum content is more preferably 90 g/L or less, still more preferably 80 g/L or less, and particularly preferably 70 g/L or less. The pH of the molybdenum fluoride aqueous solution is preferably 2 or less, and more preferably 1 or less, from the viewpoint of completely dissolving molybdenum or molybdenum oxide.

Next, the molybdenum fluoride aqueous solution is subjected to a neutralization reaction by use of an alkaline aqueous solution to obtain a fluorine-containing molybdenum hydrate cake. Here, the alkaline aqueous solution used for neutralizing the molybdenum fluoride aqueous solution is preferably aqueous ammonia with an ammonia content of 10 mass% to 30 mass%.

For the addition amount, in the neutralization step, the molar ratio of NH₃/Mo is preferably 95 or more and 500 or less, more preferably 100 or more and 450 or less, and still more preferably 110 or more and 400 or less. For the addition amount, the molar ratio of NH₃/HF is preferably 3.0 or more, more preferably 4.0 or more, and still more preferably 5.0 or more, from the viewpoint of forming a molybdenum polyoxometalate compound that is soluble in amine or dilute aqueous ammonia. On the other hand, from the viewpoint of cost reduction, the molar ratio of NH₃/HF is preferably 100 or less, more preferably 50 or less, and still more preferably 40 or less.

The addition time in the neutralization reaction is preferably 10 minutes or less, more preferably 8 minutes or less, and still more preferably 5 minutes or less.

The fluorine-containing molybdenum hydrate cake obtained by the neutralization reaction is decanted with diluted aqueous ammonia using a centrifuge, and repeatedly washed until the amount of released fluoride ions is 100 mg/L or less, whereby fluoride ions are removed from the fluorine-containing molybdenum hydrate cake to obtain a molybdenum-containing precipitate. In the fluorine-containing molybdenum hydrate cake obtained by the neutralization reaction, fluorine compounds such as ammonium fluoride are present as impurities, and are preferably removed. The cleansing liquid used for removing fluoride ions is preferably diluted aqueous ammonia.

For the molybdenum content of the molybdenum-containing precipitate obtained, a part of the molybdenum-containing precipitate is collected, dried at 110°C for 24 hours, and then fired at 1,000°C for 4 hours to form MoO₃. The weight of MoO₃ thus formed is measured. From the weight, the molybdenum content of the molybdenum-containing precipitate can be calculated.

To the molybdenum-containing precipitate obtained, a tertiary amine compound and pure water are added, and the mixture is stirred for 10 minutes to obtain a molybdenum-containing mixed liquid. Thereafter, 35 mass% hydrogen peroxide is added to the molybdenum-containing mixed liquid, and the mixture is stirred for 30 minutes to obtain a molybdenum polyoxometalate compound-containing liquid.

The tertiary amine compound is mixed such that the content of the tertiary amine compound in the molybdenum-containing mixed liquid is preferably 0.1 mass% or more and 30 mass% or less. The tertiary amine compound is preferably one or more selected from trimethylamine, triethylamine and tri-n-propylamine.

The hydrogen peroxide content of the hydrogen peroxide solution is preferably 0.5 mass% to 35 mass%. Hydrogen peroxide is added such that the molar ratio of hydrogen peroxide to molybdenum, H₂O₂/Mo, is preferably 0.6 or more and 1.5 or less, and more preferably 0.7 or more and 1.2 or less because hydrogen peroxide may decompose during mixing.

Like the method for producing a tantalum polyoxometalate compound-containing liquid, the method for producing a molybdenum polyoxometalate compound-containing liquid preferably includes the step of removing hydrogen peroxide from the obtained molybdenum polyoxometalate compound-containing liquid.

A method for producing a tungsten polyoxometalate compound-containing liquid, which is one of the elements mixed with the hafnium polyoxometalate compound-containing material of the present invention, will be described below. The description of parts overlapping the method for producing the tantalum polyoxometalate compound-containing liquid is omitted.

First, tungsten or tungsten oxide is reacted with hydrofluoric acid (HF) such as an aqueous hydrofluoric acid solution to obtain tungsten fluoride (H₂MoF₆), which is dissolved in water to obtain a tungsten fluoride aqueous solution which is an acidic metal aqueous solution.

Here, the tungsten fluoride aqueous solution is preferably adjusted by adding water (for example, pure water) so that tungsten is contained at 1 to 100 g/L in terms of WO₃. Here, the tungsten content is preferably 1 g/L or more in terms of WO₃ because a tungsten polyoxometalate compound hydrate that is easily soluble in water is obtained. From the viewpoint of productivity, the hafnium content is more preferably 10 g/L or more, and still more preferably 20 g/L or more. On the other hand, the tungsten content is preferably 100 g/L or less in terms of WO₃ because a tungsten polyoxometalate compound hydrate that is easily soluble in water is obtained. For synthesizing a tungsten polyoxometalate compound hydrate that is more reliably soluble in water, the tungsten content is more preferably 90 g/L or less, still more preferably 80 g/L or less, and particularly preferably 70 g/L or less. The pH of the tungsten fluoride aqueous solution is preferably 2 or less, and more preferably 1 or less, from the viewpoint of completely dissolving tungsten or tungsten oxide.

Next, the tungsten fluoride aqueous solution is subjected to a neutralization reaction by use of an alkaline aqueous solution to obtain a fluorine-containing tungsten hydrate cake. Here, the alkaline aqueous solution used for neutralizing the tungsten fluoride aqueous solution is preferably aqueous ammonia with an ammonia content of 10 mass% to 30 mass%.

For the addition amount, in the neutralization step, the molar ratio of NH₃/W is preferably 95 or more and 500 or less, more preferably 100 or more and 450 or less, and still more preferably 110 or more and 400 or less. For the addition amount, the molar ratio of NH₃/HF is preferably 3.0 or more, more preferably 4.0 or more, and still more preferably 5.0 or more, from the viewpoint of forming a tungsten polyoxometalate compound that is soluble in amine or dilute aqueous ammonia. On the other hand, from the viewpoint of cost reduction, the molar ratio of NH₃/HF is preferably 100 or less, more preferably 50 or less, and still more preferably 40 or less.

The addition time in the neutralization reaction is preferably 10 minutes or less, more preferably 8 minutes or less, and still more preferably 5 minutes or less.

The fluorine-containing tungsten hydrate cake obtained by the neutralization reaction is decanted with diluted aqueous ammonia using a centrifuge, and repeatedly washed until the amount of released fluoride ions is 100 mg/L or less, whereby fluoride ions are removed from the fluorine-containing tungsten hydrate cake to obtain a tungsten-containing precipitate. In the fluorine-containing tungsten hydrate cake obtained by the neutralization reaction, fluorine compounds such as ammonium fluoride are present as impurities, and are preferably removed. The cleansing liquid used for removing fluoride ions is preferably diluted aqueous ammonia.

For the tungsten content of the tungsten-containing precipitate obtained, a part of the tungsten-containing precipitate is collected, dried at 110°C for 24 hours, and then fired at 1,000°C for 4 hours to form WO₃. The weight of WO₃ thus formed is measured. From the weight, the tungsten content of the tungsten-containing precipitate can be calculated.

To the tungsten-containing precipitate obtained, a tertiary amine compound and pure water are added, and the mixture is stirred for 10 minutes to obtain a tungsten-containing mixed liquid. Thereafter, 35 mass% hydrogen peroxide is added to the tungsten-containing mixed liquid, and the mixture is stirred for 30 minutes to obtain a tungsten polyoxometalate compound-containing liquid.

The tertiary amine compound is mixed such that the content of the tertiary amine compound in the tungsten-containing mixed liquid is preferably 0.1 mass% or more and 30 mass% or less. The tertiary amine compound is preferably one or more selected from trimethylamine, triethylamine and tri-n-propylamine.

The hydrogen peroxide content of the hydrogen peroxide solution is preferably 0.5 mass% to 35 mass%. Hydrogen peroxide is added such that the molar ratio of hydrogen peroxide to tungsten, H₂O₂/W, is preferably 0.6 or more and 1.5 or less, and more preferably 0.7 or more and 1.2 or less because hydrogen peroxide may decompose during mixing.

Like the method for producing a tantalum polyoxometalate compound-containing liquid, the method for producing a tungsten polyoxometalate compound-containing liquid preferably includes the step of removing hydrogen peroxide from the obtained tungsten polyoxometalate compound-containing liquid.

A method for producing a rare earth polyoxometalate compound-containing liquid, which is one of the elements mixed with the hafnium polyoxometalate compound-containing material of the present invention, will be described below. The description of parts overlapping the method for producing the tantalum polyoxometalate compound-containing liquid is omitted.

First, rare earth or rare earth oxide is reacted with hydrofluoric acid (HF) such as an aqueous hydrofluoric acid solution to obtain rare earth fluoride, which is dissolved in water to obtain a rare earth fluoride aqueous solution which is an acidic metal aqueous solution.

Here, the rare earth fluoride aqueous solution is preferably adjusted by adding water (for example, pure water) so that rare earth is contained at 1 to 100 g/L in terms of rare earth oxide. Here, the rare earth content is preferably 1 g/L or more in terms of rare earth oxide because a rare earth polyoxometalate compound hydrate that is easily soluble in water is obtained. From the viewpoint of productivity, the hafnium content is more preferably 10 g/L or more, and still more preferably 20 g/L or more. On the other hand, the rare earth content is preferably 100 g/L or less in terms of rare earth oxide because a rare earth polyoxometalate compound hydrate that is easily soluble in water is obtained. For synthesizing a rare earth polyoxometalate compound hydrate that is more reliably soluble in water, the rare earth content is more preferably 90 g/L or less, still more preferably 80 g/L or less, and particularly preferably 70 g/L or less. The pH of the rare earth fluoride aqueous solution is preferably 2 or less, and more preferably 1 or less, from the viewpoint of completely dissolving rare earth or rare earth oxide.

Next, the rare earth fluoride aqueous solution is subjected to a neutralization reaction by use of an alkaline aqueous solution to obtain a fluorine-containing rare earth hydrate cake. Here, the alkaline aqueous solution used for neutralizing the rare earth fluoride aqueous solution is preferably aqueous ammonia with an ammonia content of 10 mass% to 30 mass%.

For the addition amount, in the neutralization step, the molar ratio of NH₃/rare earth is preferably 95 or more and 500 or less, more preferably 100 or more and 450 or less, and still more preferably 110 or more and 400 or less. For the addition amount, the molar ratio of NH₃/HF is preferably 3.0 or more, more preferably 4.0 or more, and still more preferably 5.0 or more, from the viewpoint of forming a rare earth polyoxometalate compound that is soluble in amine or dilute aqueous ammonia. On the other hand, from the viewpoint of cost reduction, the molar ratio of NH₃/HF is preferably 100 or less, more preferably 50 or less, and still more preferably 40 or less.

The addition time in the neutralization reaction is preferably 10 minutes or less, more preferably 8 minutes or less, and still more preferably 5 minutes or less.

The fluorine-containing rare earth hydrate cake obtained by the neutralization reaction is decanted with diluted aqueous ammonia using a centrifuge, and repeatedly washed until the amount of released fluoride ions is 100 mg/L or less, whereby fluoride ions are removed from the fluorine-containing rare earth hydrate cake to obtain a rare earth-containing precipitate. In the fluorine-containing rare earth hydrate cake obtained by the neutralization reaction, fluorine compounds such as ammonium fluoride are present as impurities, and are preferably removed. The cleansing liquid used for removing fluoride ions is preferably diluted aqueous ammonia.

For the rare earth content of the rare earth-containing precipitate obtained, a part of the rare earth-containing precipitate is collected, dried at 110°C for 24 hours, and then fired at 1,000°C for 4 hours to form rare earth oxide. The weight of the rare earth thus formed is measured. From the weight, the rare earth content of the rare earth-containing precipitate can be calculated.

To the rare earth-containing precipitate obtained, a tertiary amine compound and pure water are added, and the mixture is stirred for 10 minutes to obtain a rare earth-containing mixed liquid. Thereafter, 35 mass% hydrogen peroxide is added to the rare earth-containing mixed liquid, and the mixture is stirred for 30 minutes to obtain a rare earth polyoxometalate compound-containing liquid.

The tertiary amine compound is mixed such that the content of the tertiary amine compound in the rare earth-containing mixed liquid is preferably 0.1 mass% or more and 30 mass% or less. The tertiary amine compound is preferably one or more selected from trimethylamine, triethylamine and tri-n-propylamine.

The hydrogen peroxide content of the hydrogen peroxide solution is preferably 0.5 mass% to 35 mass%. Hydrogen peroxide is added such that the molar ratio of hydrogen peroxide to rare earth, H₂O₂/Hf, is preferably 0.6 or more and 1.5 or less, and more preferably 0.7 or more and 1.2 or less because hydrogen peroxide may decompose during mixing.

Like the method for producing a tantalum polyoxometalate compound-containing liquid, the method for producing a rare earth polyoxometalate compound-containing liquid preferably includes the step of removing hydrogen peroxide from the obtained rare earth polyoxometalate compound-containing liquid.

A composite hafnium polyoxometalate film of the present invention contains composite hafnium polyoxometalate particles contained in the hafnium polyoxometalate compound-containing material of the present invention.

The composite hafnium polyoxometalate film of the present invention contains composite hafnium polyoxometalate particles contained in the hafnium polyoxometalate compound-containing material of the present invention.

The method for producing a composite hafnium polyoxometalate film according to the present invention includes the step of forming a composite hafnium polyoxometalate film by applying, and drying and/or firing a composite polyoxometalate compound-containing material formed by the method for producing a composite hafnium polyoxometalate compound-containing material according to the present invention.

Like the method for producing a hafnium polyoxometalate compound dried film, a method for producing a composite hafnium polyoxometalate dried film of the present invention, among the composite hafnium polyoxometalate films of the present invention, includes an application step of applying the composite hafnium polyoxometalate compound-containing material of the present invention to a surface of a substrate, and a film drying step of preparing a dried film by drying the composite hafnium polyoxometalate compound-containing material applied to the surface of the substrate.

Like the method for producing a hafnium polyoxometalate compound fired film according to the present invention, a method for producing a composite hafnium polyoxometalate fired film of the present invention, among the composite hafnium polyoxometalate films of the present invention, includes an application step of applying the composite hafnium polyoxometalate compound-containing material of the present invention to a surface of a substrate, a film drying step of preparing a dried film by drying the composite hafnium polyoxometalate compound-containing material applied to the surface of the substrate, and a film firing step of preparing a fired film by firing the dried film at a firing temperature of 300°C or higher and 1,200°C or lower for a firing time of 1 hour or more and 12 hours or less in the atmosphere.

Unless otherwise stated, the expression "X to Y" (each of X and Y is an arbitrary number), as used in the present specification, includes the meaning of "X or more and Y or less", as well as the meaning of "preferably more than X" or the meaning of "preferably less than Y". In addition, the expression "X or more" (X is an arbitrary number) or "Y or less" (Y is an arbitrary number) includes the meaning of "preferably more than X" or "preferably less than Y".

### Advantageous Effects of Invention

The hafnium polyoxometalate compound-containing material of the present invention is basic and excellent in dispersibility.

### Brief Description of Drawings

Fig. 1 is a list of physical property values of hafnium polyoxometalate compound-containing materials of Examples 1 to 5 of the present invention and Comparative Example 1, and measurement results.
Fig. 2 is a graph that illustrates the transmittances of the hafnium polyoxometalate compound-containing materials of Examples 1 to 5 of the present invention and Comparative Example 1.
Fig. 3 is a chart that illustrates the result of measuring the XRD spectrum of the hafnium polyoxometalate compound-containing material of Example 4 of the present invention.

### Best Mode for Carrying Out the Invention

Hereinafter, the hafnium polyoxometalate compound-containing materials of embodiments according to the present invention will be described in further detail by way of the following examples. However, the following examples do not limit the present invention.

### Example 1

To 76.0 g of hafnium oxide (purity: 98%, powder, manufactured by Kojundo Chemical Lab. Co., Ltd.), 105.1 g of 55 mass% hydrofluoric acid and 796.9 g of pure water were added, and the mixture was heated to 80°C using a water bath, and stirred for 24 hours to be dissolved, thereby obtaining a hydrofluoric acid solution of a hafnium compound. A hafnium complex aqueous solution obtained by adding 2.2 g of a 35 mass% hydrogen peroxide solution to 60 g of the hydrofluoric acid solution of a hafnium compound (H₂O₂/Hf molar ratio = 1.0) was stirred for 5 minutes, and then gradually added to 377.2 g of 25 mass% aqueous ammonia (NH₃/Hf molar ratio = 250). Thereafter, the mixture was stirred for 5 minutes to obtain a neutralization reaction liquid containing hafnium hydroxide as a precipitate.

Next, the neutralization reaction liquid was decanted with a centrifuge, and a precipitate (containing hafnium hydroxide) was collected. The collected precipitate and 200 g of 25 mass% aqueous ammonia were mixed, the mixture was made into a slurry, and then decanted again, and the resulting precipitate was collected. The step of decantation and collection of a precipitate (containing hafnium hydroxide) was repeated three times.

Subsequently, 31.1 g of 25 mass% TMAH was added to the collected precipitate (containing hafnium hydroxide) to obtain a mixed solution. Pure water was added until the total weight of the mixed liquid reached 77.7 g so that the ultimate hafnium content was 6% by mass in terms of HfO₂, and the mixed liquid was stirred for 6 hours to obtain a colorless and transparent hafnium polyoxometalate compound-containing liquid of Example 1. The pH of the hafnium polyoxometalate compound-containing liquid of Example 1 was 14.8. The hafnium polyoxometalate compound-containing liquid of Example 1 was dried. In the obtained dried powder, the XRD maximum intensity (Ia) at a diffraction angle 20 of 31° or more and 33° or less was 478.333, and the XRD maximum intensity (Ib) at a diffraction angle 2θ of 5° or more and 6° or less was 1255.00. The ratio of the XRD maximum intensity (Ib) to the XRD maximum intensity (Ia), Ib/Ia, was 2.62.

### Example 2

In Example 2, a colorless and transparent hafnium polyoxometalate compound-containing liquid of Example 2 was prepared by carrying out the same production method as in Example 1 except that instead of 31.1 g of 25 mass% TMAH, 22.2 g of 35 mass% TEAH was added to the collected precipitate (containing hafnium hydroxide) to obtain a mixed liquid. The pH of the obtained hafnium polyoxometalate compound-containing liquid of Example 2 was 14.3. The hafnium polyoxometalate compound-containing liquid of Example 2 was dried. In the obtained dried powder, the XRD maximum intensity (Ia) at a diffraction angle 20 of 31° or more and 33° or less was 523.333, and the XRD maximum intensity (Ib) at a diffraction angle 2θ of 5° or more and 6° or less was 785.000. The ratio of the XRD maximum intensity (Ib) to the XRD maximum intensity (Ia), Ib/Ia, was 1.50.

### Example 3

In Example 3, a colorless and transparent hafnium polyoxometalate compound-containing liquid of Example 3 was prepared by carrying out the same production method as in Example 1 except that instead of 31.1 g of 25 mass% TMAH, 19.4 g of 40 mass% benzyltrimethylammonium hydroxide was added to the collected precipitate (containing hafnium hydroxide) to obtain a mixed liquid. The pH of the obtained hafnium polyoxometalate compound-containing liquid of Example 3 was 14.4. The hafnium polyoxometalate compound-containing liquid of Example 3 was dried. In the obtained dried powder, the XRD maximum intensity (Ia) at a diffraction angle 2θ of 31° or more and 33° or less was 440.000, and the XRD maximum intensity (Ib) at a diffraction angle 2θ of 5° or more and 6° or less was 1083.33. The ratio of the XRD maximum intensity (Ib) to the XRD maximum intensity (Ia), Ib/Ia, was 2.46.

### Example 4

In Example 4, a colorless and transparent hafnium polyoxometalate compound-containing liquid of Example 4 was prepared by carrying out the same production method as in Example 1 except that instead of 31.1 g of 25 mass% TMAH, 16.2 g of 47 to 50 mass% choline was added to the collected precipitate (containing hafnium hydroxide) to obtain a mixed liquid. The pH of the obtained hafnium polyoxometalate compound-containing liquid of Example 4 was 14.5. The hafnium polyoxometalate compound-containing liquid of Example 4 was dried. In the obtained dried powder, the XRD maximum intensity (Ia) at a diffraction angle 20 of 31° or more and 33° or less was 600.000, and the XRD maximum intensity (Ib) at a diffraction angle 2θ of 5° or more and 6° or less was 701.667. The ratio of the XRD maximum intensity (Ib) to the XRD maximum intensity (Ia), Ib/Ia, was 1.17.

### Example 5

In Example 5, a colorless and transparent hafnium polyoxometalate compound-containing liquid of Example 5 was prepared by carrying out the same production method as in Example 1 except that instead of 31.1 g of 25 mass% TMAH, 11.6 g of 47 to 50 mass% choline was added to the collected precipitate (containing hafnium hydroxide) to obtain a mixed liquid, and pure water was added so that the ultimate hafnium content was 10 mass% in terms of HfO₂. The pH of the obtained hafnium polyoxometalate compound-containing liquid of Example 5 was 14.5. The hafnium polyoxometalate compound-containing liquid of Example 5 was dried. In the obtained dried powder, the XRD maximum intensity (Ia) at a diffraction angle 2θ of 31° or more and 33° or less was 718.333, and the XRD maximum intensity (Ib) at a diffraction angle 2θ of 5° or more and 6° or less was 521.667. The ratio of the XRD maximum intensity (Ib) to the XRD maximum intensity (Ia), Ib/Ia, was 1.38.

### Comparative Example 1

A hafnium polyoxometalate compound-containing material of Comparative Example 1 was obtained through the following step.

First, 634 g of ion-exchange water was added to 333 g of hafnium hydroxide (45 wt% in terms of HfO₂) to obtain a hafnium hydroxide aqueous solution. While the obtained hafnium hydroxide aqueous solution was stirred, 33 g of 67.5 wt% nitric acid was added to the hafnium hydroxide aqueous solution, thereby obtaining a slurry. In the slurry, the hafnium content was 15 wt% in terms of HfO₂, and the nitric acid content was 0.5 gram equivalents per 1 mol of hafnium.

Next, the slurry was heated to 150°C, held for 24 hours, and then left standing to be naturally cooled, thereby obtaining a crystalline hafnia sol of Comparative Example 1. The crystalline hafnia sol of Comparative Example 1 was dried. In the obtained dried powder, the XRD maximum intensity (Ia) at a diffraction angle 2θ of 31° or more and 33° or less was 1050.00, and the XRD maximum intensity (Ib) at a diffraction angle 2θ of 5° or more and 6° or less was 418.333. The ratio of the XRD maximum intensity (Ib) to the XRD maximum intensity (Ia), Ib/Ia, was 0.40.

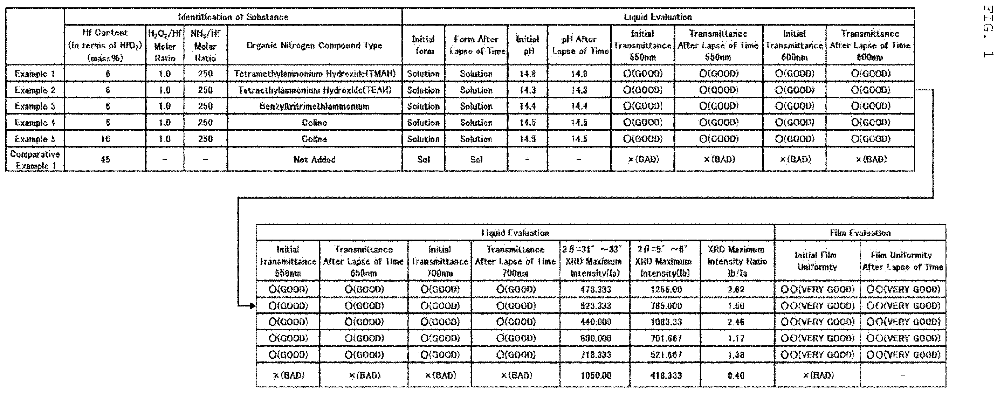

The following physical properties of the hafnium polyoxometalate compound-containing liquids of Examples 1 to 5 and the crystalline hafnia sol of Comparative Example 1 were measured. The measured physical property values and the methods for measuring the physical property values are shown below, and the measurement results are shown in Fig. 1.

### <Elemental analysis>

The sample was appropriately diluted with dilute hydrochloric acid as necessary, and the Hf weight fraction in terms of HfO₂ was measured by ICP emission spectrometry (AG-5110 manufactured by Agilent Technologies).

### <Measurement of pH>

An electrode (standard ToupH electrode 9615S-10D manufactured by HORIBA, Ltd.) of a pH meter (glass electrode type hydrogen ion concentration indicator D-51 manufactured by HORIBA, Ltd.) was immersed in the hafnium polyoxometalate compound-containing liquids of Examples 1 to 5 and the crystalline hafnia sol of Comparative Example 1, and it was confirmed that the liquid temperature had stabilized at 25°C, followed by measurement of pH. The term "initial pH" in Fig. 1 refers to the pH of a hafnium polyoxometalate compound-containing liquid adjusted to a liquid temperature of 25°C immediately it is formed. The term "pH after lapse of time" in Fig. 1 refers to the pH of a hafnium polyoxometalate compound-containing liquid after it is left standing for 1 month in a thermostat set at a room temperature of 25°C. For the crystalline hafnia sol of Comparative Example 1, it was difficult to measure the "pH after lapse of time" because crystalline hafnia particles were precipitated.

### <Measurement of transmittance>

4 ml of the hafnium polyoxometalate compound-containing liquid of each of Examples 1 to 5 was placed in a quartz cell having an optical path length of 5.0 mm, and the transmittance of the hafnium polyoxometalate compound-containing liquid of each of Examples 1 to 5 was measured at wavelengths of 550 nm, 600 nm, 650 nm and 700 nm under the above-described transmittance measurement conditions using a spectrophotometer. "O (GOOD)" was assigned when the transmittance was 70%T or more and "× (BAD)" was assigned when the transmittance was less than 70%T at wavelengths of 550 nm, 600 nm 650 nm and 700 nm. The term "initial transmittance" in Fig. 1 refers to the transmittance of a hafnium polyoxometalate compound-containing liquid adjusted to a liquid temperature of 25°C immediately after it is formed. The term "transmittance after lapse of time" in Fig. 1 refers to the transmittance of a hafnium polyoxometalate compound-containing liquid after it is left standing for 1 month in a thermostat set at a room temperature of 25°C. Further, Fig. 2 is a graph illustrating the transmittances of the hafnium polyoxometalate compound-containing liquids of Examples 1 to 5 and the crystalline hafnia sol of Comparative Example 1.

### <Measurement of XRD maximum intensity ratio>

Dried powder obtained by drying the hafnium polyoxometalate compound-containing liquid of each of Examples 1 to 5 at 150°C for 15 hours in a vacuum atmosphere was used as a sample, on which measurement was performed under the above-described X-ray powder diffraction measurement conditions. Specifically, the XRD maximum intensity (Ia) at a diffraction angle 2θ of 31° or more and 33° or less and the XRD maximum intensity (Ib) at a diffraction angle 2θ of 5° or more and 6° or less were measured. The ratio of the XRD maximum intensity (Ib) to the XRD maximum intensity (Ia), Ib/Ia, was calculated.

### <Film uniformity test>

The appearance of a coating film formed on a surface of a glass substrate used as a substitute for a current collecting plate was evaluated by observation with an optical microscope. The hafnium polyoxometalate compound-containing liquid of each of Examples 1 to 5 was dropped to a 15 mm × 15 mm glass substrate using a syringe, and applied by spin coating (1,500rpm, 30 sec). The coated portion was dried at 60°C for 30 minutes to form a coating film on the glass substrate. The formed coating film was observed with an optical microscope at a magnification of 100 times. "○ 'VERY GOOD)" was assigned when coarse particles were not present in the coating film, and none of bubbling, coating unevenness and cracking occurred, "○ (GOOD)" was assigned when coarse particles were not present in the coating film, and at least one of bubbling, coating unevenness and cracking occurred, and "× (BAD)" was assigned when coarse particles were present in the coating film, and at least one of bubbling, coating unevenness and cracking occurred. The term "initial film uniformity" in Fig. 1 refers to film uniformity of a coating film formed from a hafnium polyoxometalate compound-containing liquid immediately after it is formed. The term "film uniformity after lapse of time" in Fig. 1 refers to the film uniformity of a film formed after the hafnium polyoxometalate compound-containing liquid immediately after it is formed is left standing for 1 month in a thermostat set at a room temperature of 25°C.

As shown in Fig. 1, the hafnium polyoxometalate compound-containing liquids of Examples 1 to 5 had excellent solubility in a solvent when in dried powder obtained by drying the hafnium polyoxometalate compound-containing liquids at 150°C for 15 hours in a vacuum atmosphere, the ratio of an XRD maximum intensity (Ib) at a diffraction angle 2θ of 5° or more and 6° or less to an XRD maximum intensity (Ia) at a diffraction angle 2θ of 31° or more and 33° or less, Ib/Ia, was 1.1 or more. Here, Fig. 3 is a chart that illustrates the result of measuring the XRD spectrum of the hafnium polyoxometalate compound-containing liquid of Example 4. The pattern observed at a diffraction angle 20 of 31° or more and 33° or less corresponds to the XRD maximum intensity (Ia), and the XRD maximum intensity (Ib) at a diffraction angle 2θ of 5° or more and 6° or less corresponds to the XRD maximum intensity (Ib).

The hafnium polyoxometalate compound-containing liquids of Examples 1 to 5 had excellent dispersibility when the maximum value of transmittance in a wavelength region of 550 nm to 700 nm was 70%T or more.

The hafnium polyoxometalate compound-containing liquids of Examples 1 to 5 had good stability when the hafnium content in the hafnium polyoxometalate compound-containing material was more than 0 to 10 mass% in terms of HfO₂.

When the organic nitrogen compound contained in the hafnium polyoxometalate compound-containing liquids of Examples 1 to 5 was quaternary ammonium, in particular, one or more selected from tetramethylammonium hydroxide (TMAH), tetraethylammonium hydroxide (TEAH), choline and benzyltrimethylammonium hydroxy, not only high solubility was exhibited, but also crystallization was suppressed to a high degree and solation was suppressed to a high degree.

When the hafnium polyoxometalate compound-containing liquids of Examples 1 to 5 had a pH of more than 7, they were basic, and excellent in stability after lapse of time.

For the hafnium polyoxometalate compound-containing liquids of Examples 1 to 5, coating films formed from the hafnium polyoxometalate compound-containing liquids were observed with an optical microscope at a magnification of 100 times, and the results showed that excellent film uniformity was exhibited.

The invention disclosed herein includes, in addition to the configurations of the respective inventions and embodiments, those obtained by changing a part of these configurations to other configurations disclosed herein, those obtained by adding, to these configurations, other configurations disclosed herein, or those obtained by eliminating a part of these configurations for generalization to the extent that a part of the effects is obtained, if applicable.

### Industrial Applicability

The hafnium polyoxometalate compound-containing material according to the present invention is basic, has high dispersibility, and is suitable as a coating material or a combined material having high film adhesion and film uniformity. In addition, the hafnium polyoxometalate compound-containing material according to the present invention is excellent in storage stability, so that it is possible to suppress occurrence of defective products due to generation of precipitates as a result of a change over time. Therefore, waste can be reduced, and hence the cost of energy for disposal of waste can be reduced. Further, the hafnium polyoxometalate compound-containing material according to the present invention also ensures good formation of a coating film, so that waste can be similarly reduced and occurrence of defective products can be suppressed for coating materials covered. For these reasons, sustainable management and efficient advantages of natural resources, and decarbonization (carbon neutral) are achieved.

## Claims

1. A hafnium polyoxometalate compound-containing material comprising hafnium, an organic nitrogen compound and a solvent,
wherein a ratio of an XRD maximum intensity (Ib) at a diffraction angle 2θ of 5° or more and 6° or less to an XRD maximum intensity (Ia) at a diffraction angle 2θ of 31° or more and 33° or less, Ib/Ia, in dried powder obtained by drying the hafnium polyoxometalate compound-containing material at 150°C for 15 hours in a vacuum atmosphere is 1.1 or more.

2. A hafnium polyoxometalate compound-containing material comprising hafnium, an organic nitrogen compound, and a solvent,
wherein a maximum value of transmittance in a wavelength region of 550 nm to 700 nm is 70%T or more.

3. The hafnium polyoxometalate compound-containing material according to claim 1 or 2, wherein a hafnium content in the hafnium polyoxometalate compound-containing material is more than 0 to 10 mass% in terms of HfO₂.

4. The hafnium polyoxometalate compound-containing material according to claim 1 or 2, wherein the hafnium polyoxometalate compound-containing material contains hydrogen peroxide.

5. The hafnium polyoxometalate compound-containing material according to claim 1 or 2, wherein the hafnium polyoxometalate compound-containing material contains water.

6. The hafnium polyoxometalate compound-containing material according to claim 1 or 2, wherein the organic nitrogen compound is quaternary ammonium.

7. The hafnium polyoxometalate compound-containing material according to claim 6, wherein the quaternary ammonium is one or more selected from tetramethylammonium hydroxide (TMAH), tetraethylammonium hydroxide (TEAH), choline, and benzyltrimethylammonium hydroxy.

8. The hafnium polyoxometalate compound-containing material according to claim 1 or 2, wherein pH of the hafnium polyoxometalate compound-containing material is more than 7.

9. The hafnium polyoxometalate compound-containing material according to claim 1 or 2, wherein a maximum value of transmittance of the hafnium polyoxometalate compound-containing material in a wavelength region of 550 nm to 700 nm is 90%T or more.

10. A method for producing a hafnium polyoxometalate compound-containing material, comprising the steps of: forming a hafnium-containing precipitate by adding an acidic hafnium aqueous solution containing hafnium to an alkaline aqueous solution; and
forming a hafnium polyoxometalate compound-containing material by adding an organic nitrogen compound to a hafnium-containing precipitation slurry obtained by making the hafnium-containing precipitate into slurry.

11. Hafnium polyoxometalate compound powder comprising hafnium polyoxometalate particles contained in the hafnium polyoxometalate compound-containing material according to claim 1 or 2.

12. A method for producing hafnium polyoxometalate compound powder, comprising the step of forming hafnium oxide powder by drying and/or firing the hafnium polyoxometalate compound-containing material according to claim 1 or 2.

13. A hafnium polyoxometalate compound film comprising hafnium polyoxometalate particles contained in the hafnium polyoxometalate compound-containing material according to claim 1 or 2.

14. A method for producing a hafnium polyoxometalate compound film comprising applying, drying and/or firing the hafnium polyoxometalate compound-containing material according to claim 1 or 2.

15. A composite hafnium polyoxometalate compound-containing material comprising the hafnium polyoxometalate compound-containing material according to claim 1 or 2, and at least one element selected from the group consisting of Si, Al, Ti, Zn, Sn, Y, Ce, Ba, Sr, P, S, La, Gd, Nd, Eu, Dy, Yb, Nb, Li, Na, K, Mg, Ca, Zr, Mo, Ta, V, Ga, Ge and W.

16. A method for producing a composite hafnium polyoxometalate compound-containing material, comprising the step of forming a composite hafnium polyoxometalate compound-containing material by mixing the hafnium polyoxometalate compound-containing material formed by the method for producing a hafnium polyoxometalate compound-containing material according to claim 10 and at least one element selected from the group consisting of Si, Al, Ti, Zn, Sn, Y, Ce, Ba, Sr, P, S, La, Gd, Nd, Eu, Dy, Yb, Nb, Li, Na, K, Mg, Ca, Zr, Mo, Ta, V, Ga, Ge and W.

17. A composite hafnium polyoxometalate compound film comprising composite hafnium polyoxometalate particles contained in the composite hafnium polyoxometalate compound-containing material according to claim 15.

18. A method for producing a composite hafnium polyoxometalate compound film, comprising applying, drying and/or firing the composite hafnium polyoxometalate compound-containing material formed by the method for producing a composite hafnium polyoxometalate compound-containing material according to claim 16.
